# EUROPEAN PATENT SPECIFICATION

(11) **EP 0 739 899 B1**
(45) Date of publication and mention of the grant of the patent: **13.06.2001**
(21) Application number: 96106543.0
(22) Date of filing: 13.04.1989
(51) Int. Cl.: C07H 21/00, A61K 31/70, C07J 51/00, A61K 31/58

(54) **Novel oligoribonucleotide derivatives and application thereof to antiviral agents**
Oligoribonukleotid-Derivate und Verwendung davon als Antiviral-Arzneimittel
Dérivés d'oligoribonucléotide et leur application comme agents antiviraux

(30) Priority: 27.04.1988 JP 10494388; 06.06.1988 JP 13896688; 06.07.1988 JP 16814288; 12.09.1988 JP 22788788; 22.09.1988 JP 23848188; 02.02.1989 JP 2437289; 16.03.1989 JP 6405889
(43) Date of publication of application: 30.10.1996
(62) Divisional of application: 89303700.2
(73) Proprietor: ISIS PHARMACEUTICALS, INC., Carlsbad, CA 92008 (US)
(72) Inventor: Shibahara, Susumu, Kawasaki-ku, Kawasaki-shi, Kanagawa-ken (JP); Morisawa, Hirokazu, Kawasaki-ku, Kawasaki-shi, Kanagawa-ken (JP); Nakajima, Hideki, Ube-shi, Yamaguchi-ken (JP); Yamamoto, Naoki, Ube-shi, Yamaguchi-ken (JP); Mukai, Sachiko, Kawasaki-ku, Kawasaki-shi, Kanagawa-ken (JP)
(74) Representative: Bond, Bentley George

(56) References cited:
- EP-A- 0 169 775
- WO-A-89/03683
- EUROPEAN JOURNAL OF BIOCHEMISTRY, vol. 132, no. 1, 1983, pages 77-84, XP002013384 M.C.HAUGH ET AL.: "Analogs and Analog Inhibitors of ppp(A2'p)nA. Their Stability and Biological Activity"
- PROCEEDINGS OF THE NATIONAL ACADEMY OF SCIENCES OF USA, vol. 84, no. 21, November 1987, WASHINGTON US, pages 7706-7710, XP002013385 M.MATSUKURA ET AL.: "Phosphorothioate Analogs of Oligodeoxynucleotides : Inhibitors of Replication and Cytopathic Effects of Human Immunodeficiency Virus."
- NUCLEIC ACIDS RESEARCH, vol. 15, no. 15, 1987, OXFORD GB, pages 6131-6148, XP002013398 H.INOUE ET AL.: "Synthesis and Hybridization Studies on Two Complementary Nona(2'-O-methyl)ribonucleotides."
- E.R.KANDIMALLA: "", NUCL.ACIDS RES., , 1997, vol. 25, no. 2, pages 370 to 378

## Description

The present invention relates to novel oligoribonucleotide derivatives, which can inhibit proliferation of AIDS virus (HIV-1, HIV-2, etc.) causing AIDS (acquired immunodeficiency syndrome), to make AIDS virus avirulent, and the application of these derivatives to drugs, for example, antiviral agents such as anti-AIDS drugs or the like.

AIDS (acquired immunodeficiency syndrome) was found in 1981 and it has been clarified that the cause lies in HIV (human immunodeficiency virus, hereafter simply referred to as AIDS virus) belonging to the family retro virus. The number of patients shows a yearly increase all over the world. According to the WHO's report, the number of patients exceeded 110,000 in the world as of August 31, 1988. In Japan, 90 patients are reported (as of August 31, 1988). Furthermore, if-carriers infected with the virus but developing no AIDS yet are included, the number of patients will be more than 10,000,000 (estimated by WHO) in the world and 1048 in Japan (reported by the Ministry of Health and Welfare, as of August 31, 1988). A drug for completely curing AIDS that is a serious disease has not been found so far. 3'-Deoxy-3'-azidothymidine (hereafter simply referred to as "AZT") has been used in the clinical field as a drug for retarding the progress of AIDS and improving the clinical syndrome of the patient. However, side effect such as bone marrow impairment, etc. associated with its effectiveness are reported (cf. The New England Journal of Medicine, 317, 192-197, 1987). The mechanism of AZT is based on inhibition of the activity of reverse transcriptase possessed by the retro virus itself which is utilized, after invasion of the virus into host cells, to transcribe its genomic RNA onto DNA (Mitsuya, H., et al., Proc. Natl. Acad. Sci. USA, 82, 7096-7100, 1985) and it is thus probable that the mechanism would be related to the side effects.

On the other hand, it has been recently reported that phosphorothioate derivatives of deoxyoligonucelotides exhibit an anti-AIDS viral activity (cf. Proc. Natl. Acad. Sci. USA, 84, 7706-7710, 1987).

The AIDS virus has extremely frequent variations among its strains, in respect of its envelope protein. Therefore, it is extremely difficult to develop an effective vaccine and chemotherapeutic agents as described above are desired. However, nucleocido derivatives such as AZT conventionally used as chemotherapeutic agents exhibit anti-AIDS viral activity on one hand but on the other hand, involve serious side effects. Accordingly, it has been desired to develop drugs having minimized side effects and high practicability. For achieving the purpose, deoxyoligonucleotide phosphorothioate derivatives are at the stage of basic studies but it is desired to develop drugs having a more potent activity and provide such drugs at low costs.

As a result of extensive investigations to solve the foregoing problems, the present inventors have found that newly produced 2'-O-methyloligoribonucleotide derivatives and oligoribonucleotide derivatives could inhibit infection and proliferation of the AIDS virus to and in host-cells and are applicable as drugs for treating AIDS, and have accomplished the present invention based on this finding.

Thus, the present invention is directed to:
novel oligoribonucleotide derivatives represented by general formula (1) described below, and anti-AIDS viral compositions comprising the oligoribonucleotide derivatives as the effective ingredient; and novel oligoribonucleotide derivatives represented by general formula (2) described below and anti-AIDS viral compositions comprising the derivatives as the effective ingredient. 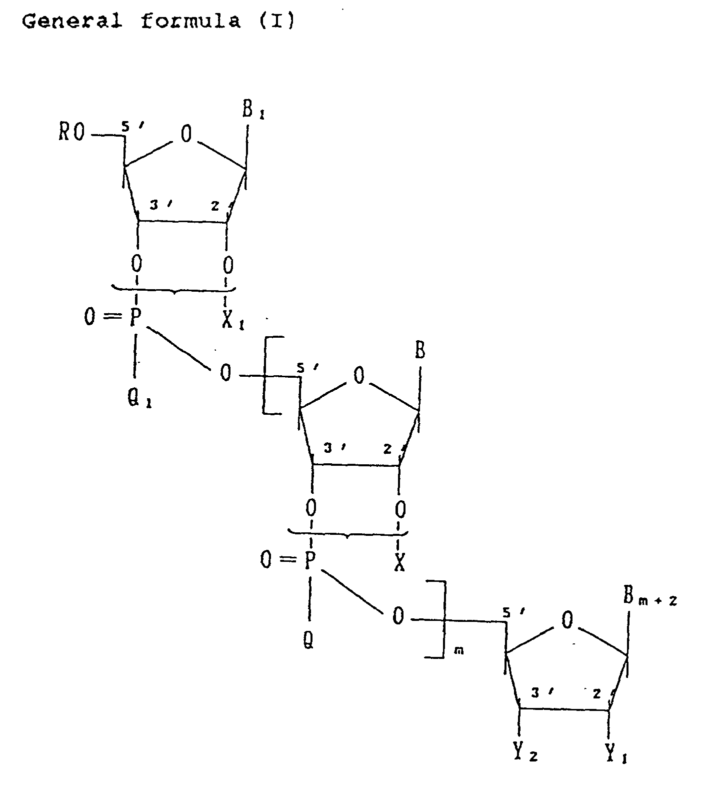
wherein:
   m is an integer of 1 to 100
   B represents B₂, B₃, ..... B₍ₘ₊₁₎ and, B₁ through B₍ₘ₊₂₎, which may be the same or different, each represents any one of hypoxanthine-9-yl, guanine-9-yl, adenine-9-yl, cytosine-1-yl, uracil-1-yl and thymine-1-yl;
   Q represents Q₂, Q₃, ..... Q₍ₘ₊₁₎ and, Q₁ through Q₍ₘ₊₁₎, independently represents any one of a thio anion, an oxo anion, an alkyl group, an aryloxy group, an alkylamino group, an arylamino group, an alkylthio group and an arylthio group (provided that at least one of Q₁ to Q₍ₘ₊₁₎ represents a thio anion);
   X represents X₂, X₃ ..... X₍ₘ₊₁₎ and, X₁ through X₍ₘ₊₁₎, which may be the same or different, each represents any one of a methyl group or an acyl group having 1 to 20 carbon atoms;
   R represents a hydrogen atom, an acyl group having 1 to 20 carbon atoms, a phosphoryl group which may optionally have a substituent, a thiophosphoryl group which may optionally have a substituent or an alkylaminophosphoryl group which may optionally have a substituent; and
   Y₁ and Y₂ independently represents methoxy group, an acyloxy group having 1 to 20 carbon atoms, a hydrogen atom, hydroxyl group, an alkyloxy group which may optionally have a substituent, a phosphoryloxy group which may optionally have a substituent, a thiophosphoryloxy group which may optionally have a substituent and an alkylaminophosphoryloxy group which may optionally have a substituent; and 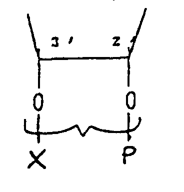 indicates that a group X is bound to an oxygen atom either at the 2' position or at the 3' position in the sugar moiety of the nucleoside monomer and that the phosphorus atom (P) is bound to an oxygen atom at either the 3' position or at the 2' position; respectively, in the sugar moiety of the nucleoside monomer, with the proviso that at least one of the groups X is bound to an oxygen atom at the 3' position.

Further in formula (1) described above, examples of the substituent on R, Y₁ and Y₂ include-cyanoethyl group, chlorophenyl group, monophosphoryl group, pyrophosphoryl group, a hydrocarbon residue having 1 to 20-carbon atoms, cholesteryl group and a group shown by J-(K-O-L)̵_{E}, which may be the same or different: provided that J represents: 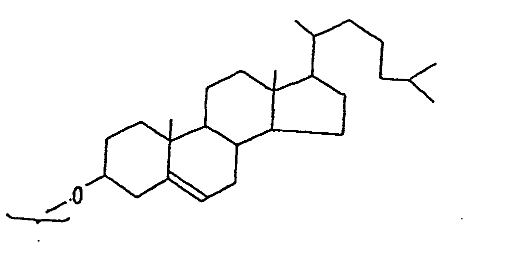 hydroxyl group or any one of the oligoribonucleotidyl group defined for the structural formula of said derivative according to General Formula (I), from which any one of RO, Y₁ and Y₂ is removed;
K represents any one of phosphoryl group, thiophosphoryl group and an alkylaminophosphoryl group having 1 to 10 carbon atoms;
L represents a hydrocarbon residue having 1 to 20 carbon atoms; and,
E represents an integer of 1 to 10.

The nucleotide sequence may not necessarily be complementary to the sequence of genomic RNA of AIDS virus or DNA integrated into chromosome.

In the formula described above, B₁ through B₍ₘ₊₂₎, Q₁ through Q₍ₘ₊₁₎ and X₁ through X₍ₘ₊₁₎ are shown from the 5'-end in the order of 1, 2, 3, 4, ... (m+1). For example, when m is 2, B₁ to B₍ₘ₊₂₎ are, in the order from the 5'-end, B₁, B₂, B₃, B₄; Q₁ to Q₍ₘ₊₁₎ are, in the order from the 5'-end, Q₁, Q₂, Q₃; and X₁ through X₍ₘ₊₁₎ are, in the order from the 5'-end, X₁, X₂, X₃; and the following structural formula is presented:

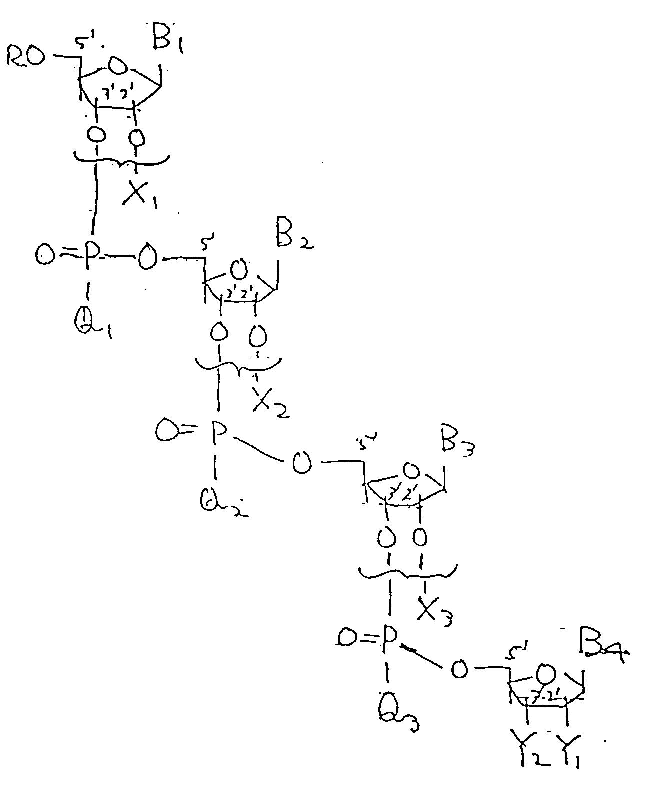

Likewise, when m is 100, B₁ to B₍ₘ₊₂₎ are, in the order from the 5'-end, B₁, B₂, B₃, ... B₁₀₀, B₁₀₁, B₁₀₂; Q₁ to Q₍ₘ₊₁₎ are, in the order from the 5'-end, Q₁, Q₂, Q₃, ... Q₁₀₀, Q₁₀₁; and X₁ through X₍ₘ₊₁₎ are, in the order from the 5'-end, X₁, X₂, X₃, ... X₁₀₀, X₁₀₁; and the following structural formula is presented: 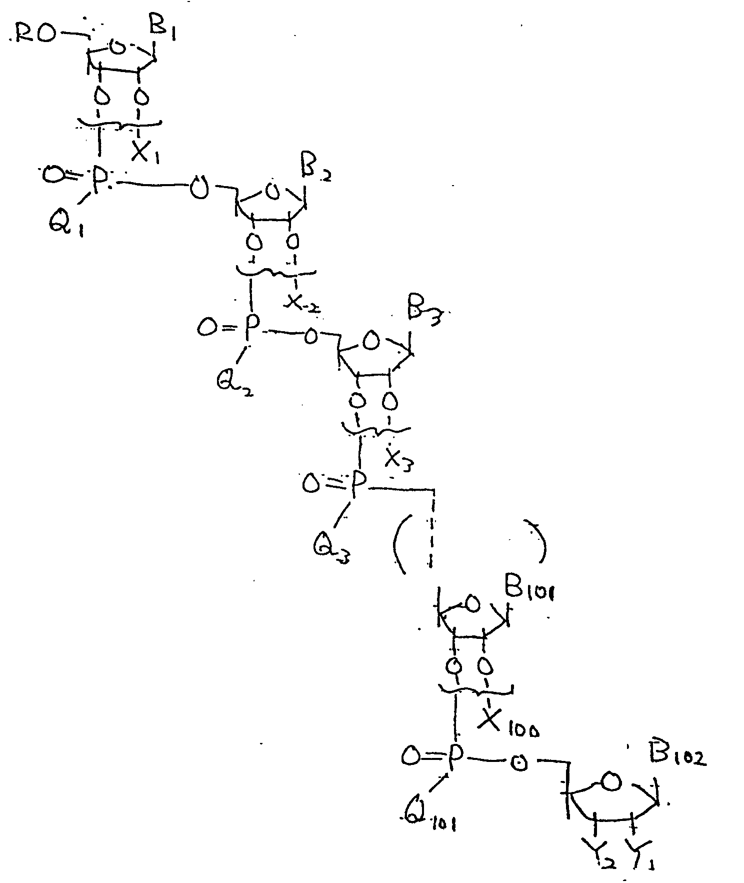 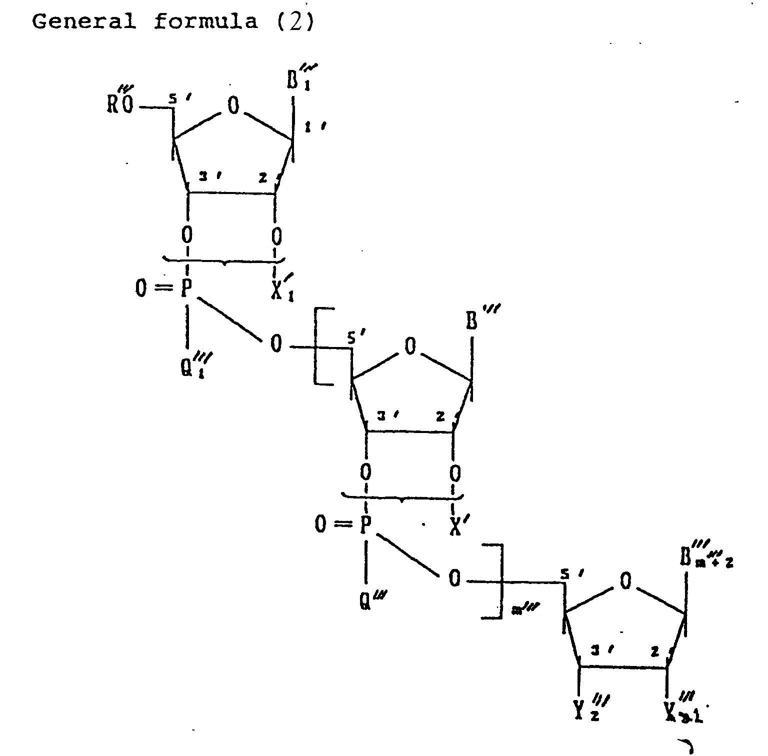 wherein:
m"' is an integer of 1 to 100;
B"' represents B"'₂, B"'₃, ..... B"'_{(m"'+1)}, provided that all of them do not represent adenine-9-yl; and, B"'₁ through B"'_{(m"'+2)}, which may be the same or different; each represents any one of hypoxanthine-9-yl, guanine-9-yl, adenine-9-yl, cytosine-1-yl, uracil-1-yl and thymine-1-yl;
Q"' represents Q"'₂, Q"'₃, ..... Q" '_{(m"'+1)} and, at least one of Q"'₁ to Q"'_{(m"'+1)} represents a thio anion. Q"'₁ through Q"'_{(m"' +1)}, independently represents any one of a thio anion, an oxo anion, an aryloxy group, an alkylamino group, an arylamino group, an alkylthio group and an arylthio group;
X' represents X'₂, X'₃, ..... X'_{(m"'+1)} and, X'₁ through X'_{(m"'+1)},
R"' represents any one of a hydrogen atom, an acyl group having 1 to 20 carbon atoms and a phosphoryl group which may optionally have a substituent;
Y"'₁ and Y"'₂ independently represents any one of methoxy group, an acyloxy group having 1 to 20 carbon atoms, an alkyloxy group which may optionally have a substituent, hydroxyl group, an alkylsilyl group, a hydrogen atom and a phosphoryl group which may optionally have a substituent.

Herein the alkyl represents a straight or branched chain hydrocarbon residue having 1 to 30 carbon atoms and the aryl represents a hydrocarbon residue having 6 to 30 carbon atoms including phenyl group. Examples of the substituent include cyanoethyl group, chlorophenyl group and a hydrocarbon residue having 1 to 20 carbon atoms.

The general formula: 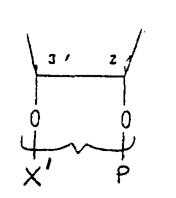 indicates that a group X' is bound to an oxygen atom either at the 2' position or at the 3' position in the sugar moiety of the nucleoside monomer and that the phosphorus atom (P) is bound to an oxygen atom at either the 3' position or at the 2' position, respectively, in the sugar moiety of the nucleoside monomer, with the proviso that at least one of the groups X' is bound to an oxygen atom at the 3' position.

The nucleotide sequence may not necessarily be complementary to the sequence of genomic RNA of AIDS virus or DNA integrated into chromosome.

Nucleotides represented by the following general formula: 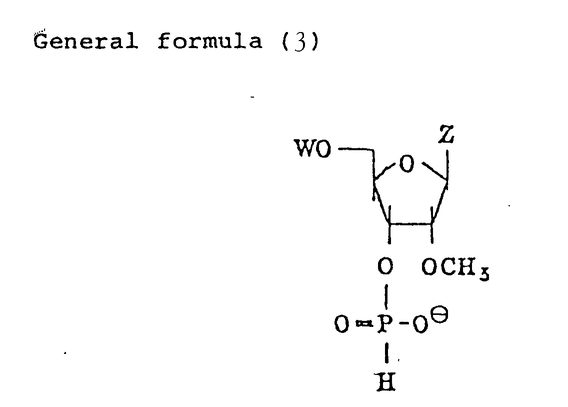 and nucleosides represented by the following general formula: 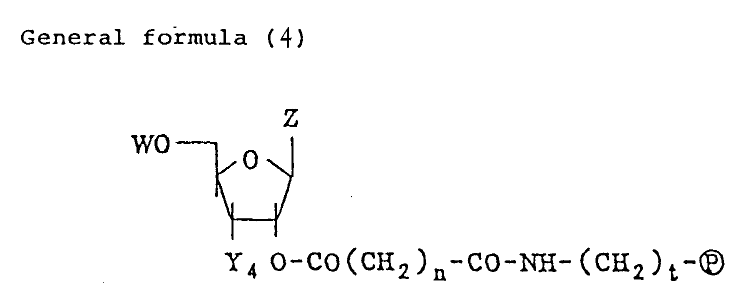 May be used as intermediates in the preparation of oligoribonucleotide derivatives in accordance with the present invention. In the general formulae (5) and (6), W represents monomethoxytrityl group or dimethoxytrityl group; Y₄ represents methoxy group, an alkyloxy group which may optionally have a substituent or a hydrogen atom; n and t, which may be different from each other, or independently, each represents an integer of 1 to 30; Ⓟ represents any one of a controlled pore glass derivative, a polystyrene derivative or a silica gel derivative; and Z represents a substituent represented by any one of general formulae below:

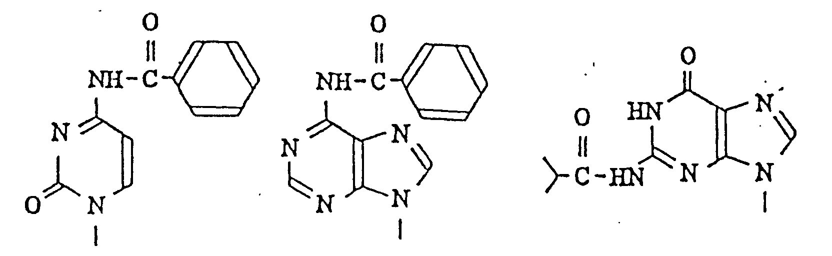

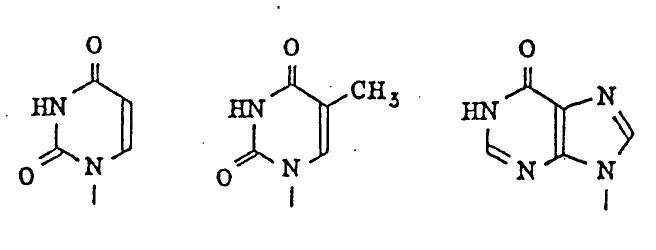

The carbon atom number of the alkyl moiety in the alkyloxy group is 1 to 10. Examples of the alkyloxy group having a substituent include: 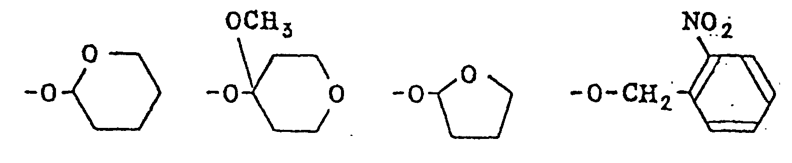 etc.

With respect to the antiviral activity of oligodeoxynucleotide, an example of using oligodeoxynucleotide for inhibiting proliferation of Raus sarcoma virus without converting it into any derivative is reported (Zamecnik, P.C., Proc. Natl. Acad. Sci. USA, 75, 280-284, 1978). On the other hand, it is reported that oligodeoxynucleotide is rapidly decomposed in culture of mammal cells, cell extracts or sera (Wickstrom, E., Journal of Biochemical and Biophysical Methods, 13, 97-102, 1986). Accordingly, in order to expect a more effective antiviral activity of oligodeoxynucleotide, it is desired that the oligodeoxynucleotide be converted into such derivatives that are not decomposed. It is reported that for example, derivatives obtained by converting the phosphodiester in oligodeoxynucleotide into methylphophonate inhibit proliferation of herpes simplex virus type I (Smith, C.C., et al., Proc. Natl. Acad. Sci. USA, 83, 2787-2791, 1986). It is also reported that derivatives obtained by converting the phosphodiester in oligodeoxynucleotide into phosphorothioate have an improved stability (Stein, C.A., Nucleic Acids Res., 16, 3209-3221, 1988). It is further reported that these derivatives inhibit proliferation of AIDS virus (Matsukura, M., et al., Proc. Natl. Acad. Sci. USA, 84, 7706-7710, 1987). It is further reported that by leading to phosphorothioate derivatives in double stranded poly RNA or an interferon inducer, antiviral activity against bovine vesicular stomatitis virus is enhanced (De Clercq, E., et al., Virology, 42, 421-428, 1970).

On the other hand, it is known that 2'-O-methylribooligonucleotide takes a structure of the 2'-hydroxy group in the sugar moiety of RNA being methylated and has the property showing resistance to various enzymes (nucleases) for decomposing DNA or RNA (Gray, M.W., et al., Biochem. Biophys. Acta, 134, 243, 1967; Dunlap, B.E., et al., Biochemistry, 10, 2581-2587, 1971). Furthermore, 2'-O-methylribooligonucleotide has the property that it forms a stable duplex with RNA having its complementary sequence and the stability is significantly better than the stability of DNA-RNA complementary double strand having the same nucleotide sequence (Inoue, H., et al., Nucleic Acids Res., 15, 6131-6148, 1987). It is also known that 2'-O-methylribooligonucleotide is used for site-specific cleavage of RNA, utilizing the property resistant to decomposition from RNase H (Inoue, H., et al., FEBS Letters, 215, 327-330, 1987). In addition, 2'-O-methylribooligonucleotide is used to produce a unidirectional deletion mutant of DNA, utilizing the property showing significant resistance to DNase Bal31 nuclease as compared to DNA (Mukai, S., et al., Nucleic Acids Symposium Series, No. 19, 117-120, 1988). The foregoing findings are based on the characteristics that 2'-O-methylribooligonucleotide forms a stable duplex with RNA having its complementary nucleotide sequence and shows resistance various enzymes (nucleases) for decomposing DNA or RNA. Moreover, it was reported in the past that poly-2'-O-methylribooligonucleotide inhibited the progress of cancer in mice caused by retro virus, while its mechanism was clearly unknown (Tennant, R.W., et al., Proc. Natl. Acad. Sci., USA, 71, 3167-3171, 1974). In addition, 2'-O-methylribooligonucleotide can be prepared as in oligoribonucleotide using less expensive raw materials than in deoxyoligonucleotide.

With respect to the structure and properties of AIDS virus, the following findings have been made according to recent studies. That is, AIDS virus is retro virus having a single stranded RNA of about 9000 nucleotides in length as the substance of gene and has reverse transcriptase. Its nucleotide sequence (primary structure) of gene is already reported (Ratner, L., et al., Nature, 313, 277-284, 1985; Muesing, M.A., et al., Nature, 313, 450-458, 1985; Sanchez-Pescador, R., et al., Science, 227, 484-492, 1985; Wain-Hobson, S., et al., Cell, 40, 9-17, 1985). Target cells of infection are helper/inducer cells which are T4 (antigen detected with a monoclonal antibody) positive as surface antigen among human lymphocytes and after infection, cause these cytopathic effects and break down. The virus after adsorption to and invasion into the cells changes to linear double stranded viral DNA by reverse transcriptase possessed by themselves utilizing tRNA^{Lys} as a primer, transfers into the nucleus to take a circular structure and is integrated into host cell chromosomal DNA to become provirus. Transcription from the provirus is effected by cell-derived RNA polymerase II as in messenger RNA (hereafter simply referred to as "mRNA") of host cells, whereby the provirus is translated into viral protein. This procedure is regulated by at least two gene products, i.e., tat (transactivator) and rev (regulator of expression of virion proteins) encoded by the virus per se. tat works as a transcription activation factor for virus upon the region called TAR element (transacting responsive element) immediately after the initiation site of transcription (Rosen, C.A., et al., Cell, 41, 813-823, 1985). The transcribed viral RNA becomes in part genome RNA encapsulated into virion and is in part utilized as mRNA for expressing gene. Several species of mRNA are present; RNA in full length for expressing viral antigen and reverse transcriptase, mRNA for expressing envelope glycoprotein which is shortened by being spliced once, mRNA which is further shortened by being spliced at least twice in order to express genes such as tat, rev, etc., and the like.

As described above, the present invention has been successful for inhibiting infection with and proliferation of AIDS virus by further derivating the phosphate moiety, taking advantages of these oligoribonucleotides. It has also been made clear that the inhibitory effect can be exhibited in any binding mode of 2'-5' bond and 3'-5' bond in the monomer of these oligomers, that is, 3'-O-methylnucleoside may be its constituent component. Accordingly, the 2'-5' bond and the 3'-5' bond in the sugar may be both contained in the monomer of the oligomer molecule. The sugar at the 3'- and 5'-termini in the oligonucleotide may optionally have an substituent as shown in general formulae (1) or (2).

As described above, the compound of the present invention is in the form that the phosphate in the oligomer is derivated. As a result of various investigations, it has been found that the derivatives containing a thiophosphate bond is particularly effective and the thiophosphate bond is not necessary for all of the phosphodiester bonds in the oligomer molecule. It has also been found that the inhibitory effect can be exhibited in any of the case that these nucleotide sequences are complementary to those of genomic RNA for AIDS virus or DNA integrated into chromosome and in the case that the nucleotide sequences are not necessary complementary.

In the present invention, the oligomer having a complementary sequence should preferably take as the target a region that plays an important role on viral gene.

The derivative (Compound XXVII) recited in claim 3 is complementary to a nucleotide sequence present downstream from the primer binding site on viral genomic RNA and, it can be expected to inhibit transcription and replication of virus.

The derivative (Compound XXVIII) recited in claim 4 is complementary to a nucleotide sequence right upstream gag gene initiation codon.

The derivative (Compound XXIX) recited in claim 5 is complementary to a nucleotide sequence in gag gene and, it can be expected to inhibit translation of gag protein.

It is noted that all these derivatives show a potent anti-AIDS viral activity, although mechanism on the activity has not been experimentally proven in detail.

On the other hand, as is described in publications (cf., Matsukura, M., et al., Proc. Natl. Acad. Sci. USA, 84, 7706-7710, 1987; Agrawal, S., et al., Proc. Natl. Acad. Sci. USA, 85, 7709-7083, 1988) regarding anti-AIDS viral activity using oligodeoxynucleotide derivatives, it is reported that oligodeoxynucleotide derivatives not complementary to the nucleotide sequence of AIDS viral genomic RNA or DNA integrated into chromosome also possess anti-AIDS viral activity, like the complementary derivatives.

On the other hand, the compounds represented by general formulae (1), and (2) in the present invention which can be produced using less expensive raw materials than in oligodeoxynucleotide derivatives exhibit the anti-AIDS viral activity, although these compounds are not necessarily complementary to the nucleotide sequence of AIDS viral genomic RNA or DNA integrated into chromosome. It has been made clear that among the compounds represented by general formula (1), for example Coumpound XXXII recited in claim 6 exhibits the anti-AIDS viral activity, although this compound is not complementary to the nucleotide sequence of AIDS viral genomic-RNA or DNA integrated into chromosome. It has also been made clear that among the compounds represented by general formula (2), for example, Compound XXVI recited in claim 10 exhibits the anti-AIDS viral activity, although this compound is not complementary to the nucleotide sequence of AIDS viral genomic RNA or DNA integrated into chromosome.

On the other hand, the compounds in the present invention have low molecular weights unlike high molecular weight double stranded RNA known as a potent interferon inducer (cf., Lanpson, G.P., et al., Proc. Natl. Acad. Sci. USA., 58, 782, 1967; Field, A.K., et al., Proc. Natl. Acad. Sci. USA., 58, 1004, 1967) and derivatives thereof (De Clercq, E., et al., Virology, 42, 421-428, 1970). The compounds in the present invention are also clearly distinguished over 2'-5' oligoA all composed of adenosines and containing 2'-5' bond which is a protein synthesis inhibitor found in cells treated with interferon (cf., Kerr, I.M., Proc. Natl. Acad. Sci. USA., 75, 256, 1978) and derivatives thereof.

The oligoribonucleotide derivatives of the present invention can be produced by known methods such as the amidite method (cf., Matteucci, M.D., et al., Tetrahedron Lett., 22, 719, 1980), the H-phosphonate method (cf., Froehler, B.C. et al. Tetrahedron Lett., 27, 469, 1986; Garegg, P.J., et al., Tetrahedron Lett., 27, 4055, 1986) and the like. 2'-O-methylribonucleotide derivatives represented by general formula (3) which can be provided as raw materials for producing 2'-O-methylribooligonucleotide derivatives according to the H-phosphonate method can be produced by leading to the protected 2'-O-methylribonucleoside derivatives by known methods (cf., Robins, M.J., et al., J. Org. Chem., 39, 1891, 1974; Heikkila, J., et al., Acta Chem. Scand., B36, 715, 1982; Inoue, H., et al., Nucleic Acids Research, 15, 6131, 1987) and then reacting with phosphorus trichloride by known methods (cf., Froehler, B.C., et al., Tetrahedron Letters, 27, 469-472, 1986; Garegg, P.J., et al., Tetrahedron Letters, 27, 4051-4054, 1986). According to the process, Compounds I, II, III, IV and V shown below were produced. 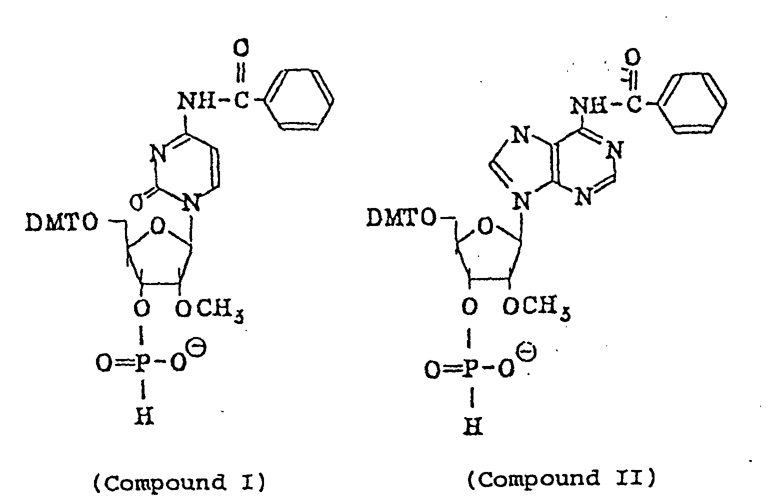 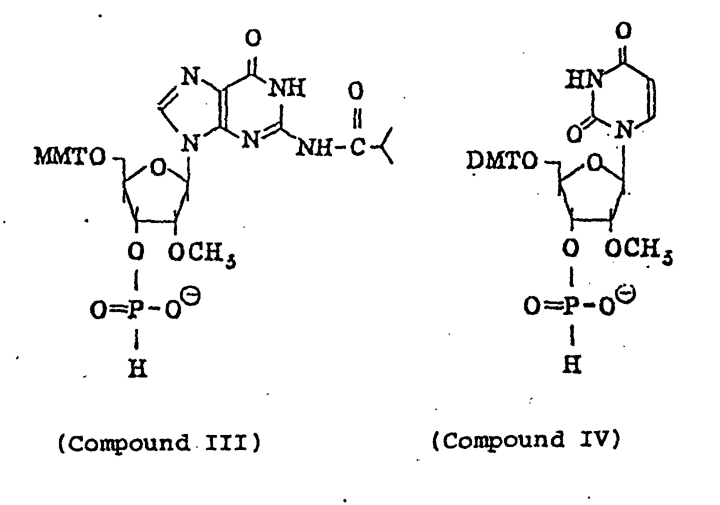 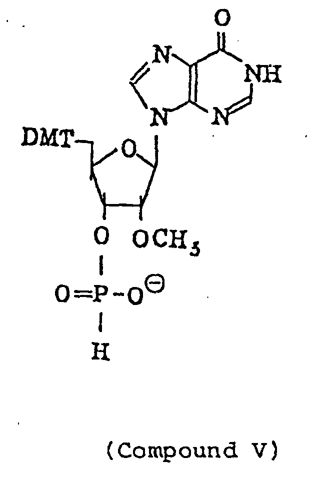 wherein DMT and MMT represent dimethoxytrityl group and monomethoxytrityl group, respectively.

Further 2'-O-methylribonucleotide derivatives represented by general formula (3) can also be produced by reacting protected 2'-O-methylribonucleoside derivatives with phosphorous acid in the presence of a condensation reagent such as triisopropylbenzenesulfonyl chloride or pivaloyl chloride by known methods (cf., Hata, T., et al., J. Am. Chem. Soc., 96, 7363, 1974; Sekine, M., et al., Tetrahedron Letters, 29, 1037-1040, 1988). Furthermore, protected 3'-O-methyl-nucleoside derivatives are produced according to known methods (cf., Robins, M.J., et al., J. Org. Chem., 39, 1891, 1974; Heikkila, J., et al., Acta Chem. Scand., B36, 715, 1982) and the derivatives can be led to 3'-O-methylnucleoside-H-phosphonate derivatives by the process described above, which can be provided as raw materials for producing the oligoribonucleotide derivatives represented by general formulae (1) and (2).

The novel nucleoside derivatives represented by general formula (4) can be produced, for example, by a known method (cf., Miyoshi, K., et al., Nucleic Acids Res., 8, 5491, 1980) using protected 3'-O-methyl (3'-O-alkyl or 3'-deoxy)nucleosides.

Using the protected novel 2'-O-methylribonucleotide derivatives, protected 3'-O-methylribonucleotide derivatives, and novel nucleoside derivatives produced by the foregoing processes, the and oligoribonucleotide derivatives can be produced, for example, by known methods (cf., Froehler, B.C., et al., Tetrahedron Letters, 27, 469-472, 1986; Garegg, P.J., et al., Tetrahedron Letters, 27, 4051-4054, 1986). That is, the nucleotide derivative elements are sequentially condensed novel nucleoside derivatives as the polymer support, having acyl chloride to elongate the chain. Then, oxidation is performed using various oxidizing agents such as sulfur (S₈), iodine (I₂) or an alkylamine/carbon tetrachloride, etc. followed by removal of the protective group and purification.

Further in the compounds represented by general formulae (1) and (2) wherein the substituents shown by Q, Q' and Q"' are thio anions or thio anions and oxo anions, these compounds can also be produced by the amidite method (cf., Matteucci, M.D., et al., Tetrahedron Lett., 22, 719, 1980; Shibahara, S., et al., Nucleic Acids Res., 15, 4403, 1987; Usman, N., et al., J. Am. Chem. Soc., 109, 7845, 1987). In this case, the monomer may be oxidized with (S₈) or iodine (I₂) in the oxidation step after the condensation and then subjected to chain elongation followed by removal of the protective group and purification.

Further in the compounds which contain at the 5'-terminal thereof, for example, an alkyl-substituted thiophosphoryl group, an alkyl-substituted phosphoryl group or an alkyl-substituted alkylaminophosphoryl group, the chain elongation of the oligomer is effected on the polymer support by the process described above; after completion of the oxidation, the oxidation product is condensed with an alkyl-H-phosphonate and the condensation product is oxidized with sulfur (S₈), iodine (I₂) or an alkylamine/carbon tetrachloride followed by removal of the protective group and purification. The derivatives in which cholesterol is bound at the 5'-terminal via a spacer can be likewise obtained by condensing with mono(monomethoxytritylated) alkanediol H-phosphonate, treating with an acid, condensing with cholesteryl-H-phosphonate, oxidizing, removing the protective group and purification.

On the other hand, the compounds containing, for example, a hydroxyalkylphosphoryloxy group or a hydroxyalkylthiophosphoryloxy group at the 3'-terminal thereof can be obtained by condensing, oxidizing, removing the protective group and purification in a similar manner, using as a starting material a polymer support having bound thereto an alkanediol monosuccinate.

The anti-AIDS viral activity can be determined by the method discribed in a publication (cf., Harada, S., et al., Science, 229, 563-566, 1985), using HTLV-III_{B} (cf., Popovic, M., et al., Science, 224, 497-500, 1984) which is an isolated strain of AIDS virus. According to this method, MT-4 cells or HTLV-I positive cell line is used as the target cell so that this method is highly sensitive to viral infection and can exhibit the cytopathic effect highly efficiently.

The novel oligoribonucleotide derivatives in accordance with the present invention have the anti-AIDS viral activity and are expected to use as anti-AIDS drugs.

The antiviral agent of the present invention may be used in a range of 0.01 to 2,000 mg, preferably 0.02 to 500 mg.

The effective component may be used in the form of, for example, a capsule, an elixir, a microcapsule or a suspension.

The compound used as the effective component of the present invention can be administered to the patient who requires treatment or prophylaxis, in a dose of 0.01 to 1,000 mg per person, generally several times in a daily dose of 0.02 to 2,000 mg. The dose can be varied depending upon condition of disease, body weight of the patient and other factors recognized by one skilled in the art.

The compound used in the present invention or physiologically acceptable salt compound or a mixture thereof can be mixed in an amount of approximately 0.2 to 500 mg together with vehicles, carriers, diluents, binders, antiseptics, stabilizers, flavors, etc. in a unit dose form required to make pharmaceutical preparations generally admitted. An amount of the active substance in these compositions or preparations is incorporated to give an appropriate dose in a range indicated.

Specific examples of drugs which can be incorporated into tablets, capsules, etc. are given below: binders such as tragacanth, gum arabic, corn starch or gelatin; excipients such as fine crystalline cellulose; swelling agents such as corn starch, pregelatinized starch, alginic acid, etc.; lubricants such as magnesium stearate; sweeteners such as sucrose, lactose or saccharine; flavors such as peppermint, Gaultheria adenothrix oil or cherry, etc. In case that the preparation unit form is a capsule, a liquid carrier such as oils and fats can additionally be incorporated into the materials of type described above. A variety of other materials may be incorporated as protectives or in order to change physical form of the preparation unit by another method. For example, tablets can be coated with shellac or sugar or with both of them. Syrup or elixir may contain sucrose as a sweetener, methyl- and propylparabens as antiseptics and pigments and flavors such as cherry or orange flavor.

In the case of enteric coating, for example, 8% aqueous solution of hydroxyphenylmethyl cellulose is used as a coating pretreatment agent, 10% aqueous solution of hydroxypropylmethyl cellulose phthalate and 3% aqueous solution of polyacetyne are used as coating agents. They are used respectively to make enteric coating preparations in a conventional manner.

A sterile composition for injection can be formulated in a conventional manner to prepare pharmaceutical preparations by dissolving or suspending the active substance, naturally occurring plant oils such as sesame oil, coconut oil, peanut oil, cotton seed oil, etc. or synthetic fatty vehicle such as ethyl oleate in a vehicle such as water for injection. Buffer agents, antiseptics, antioxidants, etc. may be added, if necessary.

### Examples

Hereafter the present invention is concretely described by referring to the examples below.

### Example 1 Production of N⁴-benzoyl-5'-O-dimethoxytrityl-2'-O-methylcytidine-3'-O-(H-phosphonate) (Compound I):

To 50 ml of a solution of 436 µl (5 mmols) of phosphorus trichloride and 5.5 ml (50 mmols) of N-methylmorpholine in dichloromethane was added 1.197 g (17.3 mmols) of 1,2,4-triazole. The mixture was stirred at room temperature for 30 minutes. To the mixture was added 17 ml of a solution of 664.7 mg (1 mmol) of N⁴-benzoyl-5'-O-dimethoxytrityl-2'-O-methylcytidine in dichloromethane over 10 minutes. The mixture was further stirred at room temperature, whereby the progress of the reaction was monitored by thin layer chromatography (mobile phase: dichloromethane/2% triethylamine/8% methanol) using silica gel 60. After it was confirmed that the raw material spot (Rf value: 0.60) disappeared and a new spot derived from Compound I appeared at Rf value of 0.41, 40 ml of triethylamine-bicarbonate buffer (1 M, pH 7.5) was added. The aqueous layer was extracted with 15 ml of dichloromethane. The dichloromethane layers were combined, dried over anhydrous sodium sulfate and then evaporated to dryness. Next, the resulting foamy residue was dissolved in 3 ml of dichloromethane containing 2% triethylamine and the solution was purified by passing through a column packed with 24 g of silica gel 60. That is, elution was carried out using 100 ml of dichloromethane/2% triethylamine, then 200 ml of dichloromethane/2% triethylamine/3% methanol and further 200 ml of dichloromethane/2% triethylamine as the mobile phase and the pure fraction of Compound I obtained was evaporated to dryness. The resulting residue was dissolved in 40 ml of dichloromethane and the solution was washed with 15 ml of 1 M triethylamine-bicarbonate buffer (pH 7.5). After drying over anhydrous sodium sulfate, the dichloromethane layer was evaporated to dryness to give 703 mg (0.849 mmol) of Compound I in yield of 84.9%.

In a manner similar to the operation described above, N⁶-benzoyl-5'-O-dimethoxytrityl-2'-O-methyladenosine-3'-O-(H-phosphonate) (Compound II), N²-isobutylyl-5'-O-monomethoxy-trityl-2'-O-methylguanosine-3'-O-(H-phosphonate) (Compound III), 5'-O-dimethoxytrityl-2'-O-methyluridine-3'-O-(H-phosphonate) (Compound IV), 5'-O-dimethoxytrityl-2'-O-methylinosine-3'-O-(H-phosphonate) (Compound V) were obtained in yields of 93.9%, 49.4%, 77.4% and 56.0%, respectively.

Next, ³¹P-NMR of these compounds was measured. With respect to Compounds I, II and III, a part of each compound was dissolved in dichloromethane and after mixing the solution with an equimolar amount of 1,8-diazabicyclo[5.4.0]undec-7-ene (simply referred to as DBU), the mixture was dropped into n-hexane to form the DBU salt, the salt was ground into powders and the powders were measured. On the other hand, Compounds IV and V were measured as they were, i.e., as the triethylamine salts. As a solvent for the measurement, d₅ pyridine was used. D₂O solution of phosphoric acid was used as the external standard. Their chemical shifts (ppm) were as follows.

| | |
|---|---|
| Compound I | -1.79 ppm |
| Compound II | -2.20 ppm |
| Compound III | -2.04 ppm |
| Compound IV | -0.73 ppm |
| Compound V | -0.40 ppm |

### Example 2 Production of 5'-O-monomethoxytrityl-N²-isobuty|yl-3'-O-methylguanosine-bound controlled pore glass:

5'-O-monomethoxytrityl-N²-isobutylyl-3'-O-methylguanosine synthesized in a conventional manner was led to 2'-O-succinylpentachlorophenyl ester by a known process (cf., Miyoshi, K., et al., Nucleic Acids Res., 8, 5491, 1980). That is, 639 mg (1 mmol) of 5'-O-monomethoxytrityl-N²-isobutylyl-3'-O-methylguanosine and 183 mg of 4-N,N-dimethylaminopyridine (merely referred to as DMAP) were dissolved in 4 ml of absolute pyridine. While stirring at room temperature, 150 mg (1.5 mmol) of succinic anhydride was added to the solution. After stirring for 90 minutes, the reaction liquid was distilled under reduced pressure. The resulting oily residue was dissolved in 35 ml of chloroform. The solution was washed 3 times with 30 ml of 0.1 M triethylamine-bicarbonate buffer (pH 7.5). After drying over anhydrous sodium sulfate, the chloroform layer was evaporated to dryness under reduced pressure. The obtained residue was dissolved in 5 ml of dimethylformamide (DMF) and 400 mg (1.5 mmol) of pentachlorophenol and 309 mg (1.5 mmol) of dicyclohexylcarbodiimide were added to the solution followed by stirring at room temperature for 13.5 hours. The precipitated insoluble matters were removed by filtration and the filtrate was removed by distillation under reduced pressure. The resulting residue was dissolved in 3 ml of chloroform and the solution was purified by column chromatography using a column packed with 20 g of silica gel 60. That is, elution was carried out using as the mobile phase, in sequence, 100 ml of chloroform, 100 ml of chloroform/0.3% methanol, 100 ml of chloroform/0.5% methanol and further 100 ml of chloroform/0.6% methanol and the resulting pure fractions of 5'-O-monomethoxytrityl-N²-isobutylyl-3'-O-methylguanosine-2'-O-succinyl-pentachlorophenyl ester were collected and evaporated to dryness under reduced pressure to give 530 mg of the ester in yield of 53.6% (Rf value: 0.73 when developed with chloroform : methanol = 20 : 1 in silica gel 60 thin layer chromatography). Next, the whole amount (530 mg) of the ester was dissolved in 5.4 ml of DMF; on the other hand, 1 g of controlled pore glass (CPG/long chain alkylamine, pore diameter: 500 Å, particle size: 122-177 µ, NH₂-: 30 µmols/g, manufactured by Pierce Chemical Inc.) was charged in a reactor equipped with a glass filter and washed with 1 ml of DMF followed by drying for a minute,in an argon gas flow. The aforesaid pentachlorophenyl ester DMF solution, 5.4 ml, and 73 µl of triethylamine were added to the pore glass, respectively. After sealing, the reaction was carried out at 30°C overnight. The reaction liquid was filtered under an argon pressure. After washing 3 times with 5 ml of DMF and then 3 times with 5 ml of tetrahydrofuran (THF), the controlled pore glass was dried for a minute in an argon flow. Next, a liquid mixture of 410 mg of DMAP, 1.4 g of 2,6-lutidine, 660 µl of acetic anhydride and 6 ml of THF was added to the pore glass. The mixture was shaken at 30°C for 15 minutes to acetylate the unreacted amino group. After filtration, the reaction mixture was washed 3 times with 5 ml of THF and then 3 times with 5 ml of diethyl ether and dried in an argon flow to give 1 g of 5'-O-monomethoxytrityl-N²-isobutylyl-3'-O-methylguanosine-bound controlled pore glass. A small amount of the product was weighed and the monomethoxytrityl group was cleaved with 3% trichloroacetic acid. The isolated tritanol was subjected to quantitative colorimetry with a perchloric acid-ethanol mixture. As the result, the bound amount was 26 µmols/g.

Furthermore, 5'-O-dimethoxytrityl-N⁶-benzoyl-3'-O-methyladenosine-bound controlled pore glass, 5'-O-dimethoxytrityl-3'-O-methylinosine-bound controlled pore glass, 5'-O-dimethoxytrityl-3'-O-methyluridine-bound controlled pore glass and 5'-O-dimethoxytrityl-N⁴-benzoyl-3'-O-methylcytidine-bound controlled pore glass were produced, respectively, in a manner similar to the above.

### Example 3 Production of Compounds XXVII, XXVIII and XXIX. 5' Amp Cmp Amp Cmp Cmp Cmp Amp Amp Ump Ump Cmp Ump Gmp Amp Amp Amp Amp Ump Gmp Gm' 3' (Compound VI), wherein m represents 2'-O-methylnucleoside unit; m' represents 3'-O-methylnucleoside unit; and P represents:

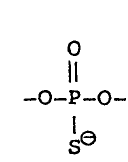

(for reference only) was produced as follows:
N²-Isobutylyl-5'-O-monomethoxytrityl-3'-O-methylguanosine-bound polymer support, 40 mg (binding amount: 1.0 µmol), was packed in a cartridge and the cartridge was set in a DNA synthesizer (model 380A, manufactured by Applied Biosystems Inc.). Following the procedure shown in Table 1, 2'-O-methylribonucleoside-3'-O-(H-phosphonate) compounds (Compounds I through IV) were treated using III, IV, II, II, II, II, III, IV, I, IV, IV, II, II, I, I, I, II, I and II in this order. After repeating 19 cycles, the intermediate for preparing the polymer support was withdrawn from the cartridge and shifted to a reactor equipped with a glass filter. After washing with 1 ml of acetonitrile, the intermediate was dried in an argon gas flow. Next, 2 ml of a solution of 0.2 M sulfur (S₈) in triethylamine : carbon disulfide : pyridine (1 : 12 : 12, volume ratio) was added to the intermediate. After shaking, the mixture was allowed to stand for 12 hours. The reaction mixture was filtered and the intermediate for synthesizing the polymer support was washed 5 times with 2 ml of carbon disulfide and once with 2 ml of diethyl ether. After drying in an argon flow, the intermediate was transferred to a glass-made bial of a 3 ml volume. 28% Ammonia water, 2 ml, was charged in the bial and sealed followed by heating at 55°C for 5 hours. The polymer support was removed by filtration and the reaction liquid was evaporated to dryness under reduced pressure. The resulting residue was dissolved in 300 µl of 0.1 M triethyl ammonium-acetic acid buffer (pH 7.0, hereafter simply referred to as TEAA) containing 10% acetonitrile. The solution was purified through a column having a diameter of 1 cm and a length of 12 cm, packed with reverse phase silica gel (Waters Co., Ltd., Prep PAK-500/C-18). That is, 0.1 M TEAA buffer (pH 7.0) having a linear gradient of 10% to 35% acetonitrile was used as the mobile phase and quantitative determination was performed at absorbancy at a wavelength of 254 nm to give the fraction of Compound VI having a dimethoxytrityl group at the 5'-terminal. After the solvent was distilled off under reduced pressure, the residue was co-evaporated together with 2 ml of water 3 times and 3 ml of 80% acetic acid was added thereto followed by stirring for 10 minutes. After the reaction liquid was evaporated to dryness, the residue was further co-evaporated together with water to remove acetic acid. To the residue was added 1.5 ml of 0.1 M triethylamine-bicarbonate buffer (pH 7.5) containing 10% acetonitrile. The mixture was washed twice with 1.5 ml of diethyl ether. The aqueous layer was evaporated to dryness and 200 µl of 0.1 M TEAA buffer (pH 7.0) containing 10% acetonitrile was added to the resulting residue. After passing through a 0.45 µ filter (manufactured by Millipore Corp.), purification was performed by high performance liquid chromatography. That is, YMC Pack ODS (manufactured by Yamamura Science Co., Ltd.) column was used and 0.1 M TEAA buffer (pH 7.0) having a linear gradient. of 10% to 70% acetonitrile was used as the mobile phase. The main peak eluted at a flow rate of 1.2 ml/min was fractionated to give 52.9 OD^{260 nm} units (about 226 nmols) of Compound VI in yield of 22.6%.

Compound VI was dissolved in D₂O containing 10 mM triethylamine-bicarbonate buffer (pH 7.5) and ³¹P-NMR was measaured (device for measurement: JEOL JMS-DX300, manufactured by JEOL Inc.). When trimethyl phosphate was used as the external standarad, Compound VI showed a characteristic signal in multiplet phosphorothioate at 51-55 ppm.

**Table 1**

| One Cycle of Procedure for Chain Elongation Reaction | | | |
|---|---|---|---|
| Operation Number | Name of Reagent or Operation | Liquid Feeding Time (second) | Number of Time |
| 1. | Washing with acetonitrile | 30 | 1 |
| | | | |
| 2. | Drying in argon | 20 | 1 |
| | | | |
| 3. | 3% Trichloroacetic acid/ dichloromethane | 30 | 6 |
| | | | |
| 4. | Drying in argon | 5 | 1 |
| | | | |
| 5. | Washing with acetonitrile Drying in argon | 30+60 5+20 | 2 |
| | | | |
| 6. | 0.2 M Compounds I-V, XV-XIII, XXI or XXII/acetonitrile 1·0 M Pivaloyl chloride/pyridine | 13 | 1 |
| | | | |
| 7. | Allowing to stand | 180 | 1 |
| | | | |
| 8. | Drying in argon | 15 | 1 |
| | | | |
| 9. | Washing with acetonitrile Drying in argon | 30 20 | 3 |

In a similar manner, Compounds XXVII, XXVIII and XXIX were produced.

### Example 4 Production of Compounds XXVI and XXXII.Compound XXIII (for reference only) was prepared as follows:

5'-O-Dimethoxytrityl-2'-O-(tert-butyldimethylsilyl)inosine-CPG (controlled pore glass), 40 mg (binding amount: 1.0 µmol) each, was packed in 3 cartridges and the cartridges were set in a DNA synthesizer (model 380A, manufactured by Applied Biosystems Inc.). Following the procedure shown in Table 1, 5'-O-dimethoxytrityl-2'-O-(tert-butyldimethylsilyl)inosine-3'-O-(H-phosphonate) (Compound XXI) was used together with pivaloyl chloride. After repeating 19 cycles, the intermediate for preparing the polymer support was withdrawn from each cartridge and shifted to a reactor equipped with a glass filter. After washing with 1 ml of acetonitrile, the intermediate was dried in an argon gas flow. Next, 2 ml of a solution of 0.2 M sulfur (S₈) in triethylamine : carbon disulfide : pyridine (1 : 12 : 12, volume ratio) was added to the intermediate. After shaking, the mixture was allowed to stand for 2 hours. The reaction liquid was filtered and the intermediate for synthesizing the polymer support was washed 5 times with 2 ml of carbon disulfide and once with 2 ml of diethyl ether. After drying in an argon flow, the intermediate was transferred to a glass-made bial of a 3 ml volume. 28% Ammonia water, 1.5 ml, and 0.5 ml of ethanol were charged in the bial and sealed followed by heating at 55°C for 5 hours. The polymer support was removed by filtration and washed with 1 ml of ethanol and then with 1 ml of 50% ethanolic water. The filtrates were combined and evaporated to dryness. To the resulting residue was added 1.5 ml of THF solution (1 M) of tetra-(n-butyl)ammonium fluoride. The mixture was shaken at room temperature for 17 hours. Next, 1 ml of 20 mM triethyl ammonium-bicarbonate buffer (pH 7.0, hereafter simply referred to as TEAB) to dilute. The dilution was purified by gel filtration chromatography using a Sephadex G25-packed column (diameter of 1.6 cm, length of 5 cm, NAP-25, manufactured by Pharmacia Inc.). That is, after the reaction dilution described above was added, elution was performed using 20 mM TEAB buffer as the mobile phase and passed-through fractions were collected and evaporated to dryness. The residue was dissolved in 300 µl of 0.1 M triethylamine-acetate buffer (pH 7.0, hereafter simply referred to as TEAA) containing 10% acetonitrile and the solution was purified through a column packed with reverse phase silica gel (Waters Co., Ltd., Prep PAK-500/C-18). That is, 0.1 M TEAA buffer having a linear gradient of 10% to 40% acetonitrile was used as the mobile phase and quantitative determination was performed at absorbancy at a wavelength of 254 nm to give the fraction of Compound XXIII having a dimethoxytrityl group at the 5'-terminal. After the solvent was distilled off, the residue was co-evaporated together with water 3 times and 3 ml of 80% acetic acid was added thereto followed by stirring for 10 minutes. After the reaction liquid was evaporated to dryness, the residue was further co-evaporated under reduced pressure together with water to remove acetic acid. To the residue was added 1.5 ml of 20 mM TEAB buffer. The mixture was washed twice with 1.5 ml of ethyl acetate. The aqueous layer was evaporated to dryness and 200 µl of 0.1 M TEAA buffer containing 10% acetonitrile was added to the resulting residue. The mixture was purified by high performance liquid chromatography. That is, YMC Pack ODS (manufactured by Yamamura Science Co., Ltd.; diameter of 6 mm, length of 30 cm) column was used and 0.1 M TEAA having a linear gradient of 10% to 50% acetonitrile was used as the mobile phase. The main peak eluted at a flow rate of 1.2 ml/min was fractionated to give 126 OD^{260 nm} units (about 514 nmols) of Compound XXIII in yield of 12.9%.

Furthermore, Compounds XXV (for reference only - shown below) and XXVI, were produced in a manner similar to the procedure described above.

With respect to Compound XXXII, the compound was produced by treating Compound XXV with acetic anhydride and pyridine.

Compound XXV is a derivative having the structural formula: 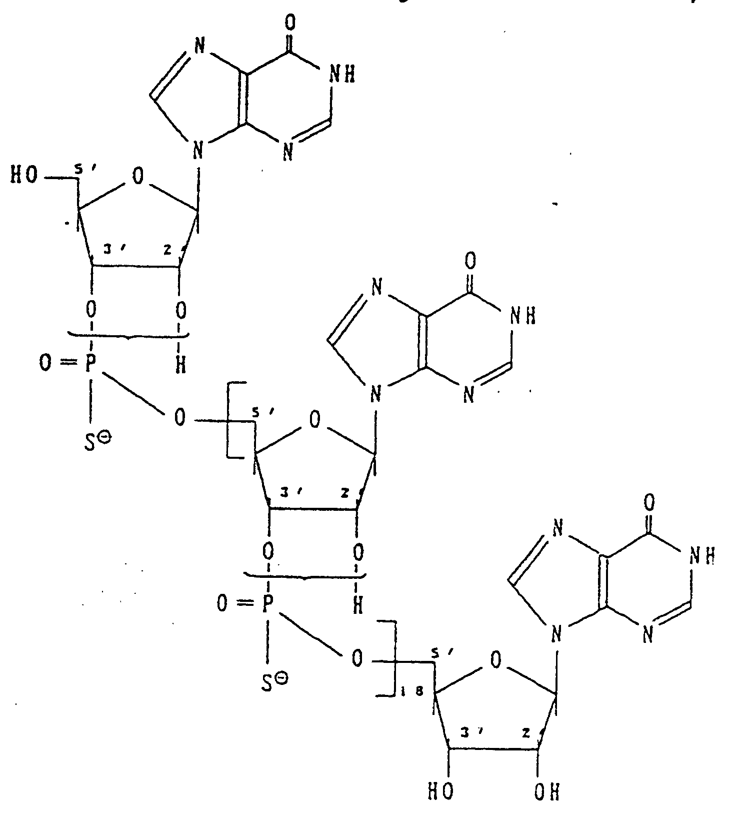 wherein a hydrogen atom (H) is capable of binding to an oxygen atom either at the 2'-position or at the 3'-position in the sugar moiety of the nucleoside monomer and, in this case, another is bound to phosphorus atom (P).

## Claims

1. An oligoribonucleotide derivative represented by the general formula: 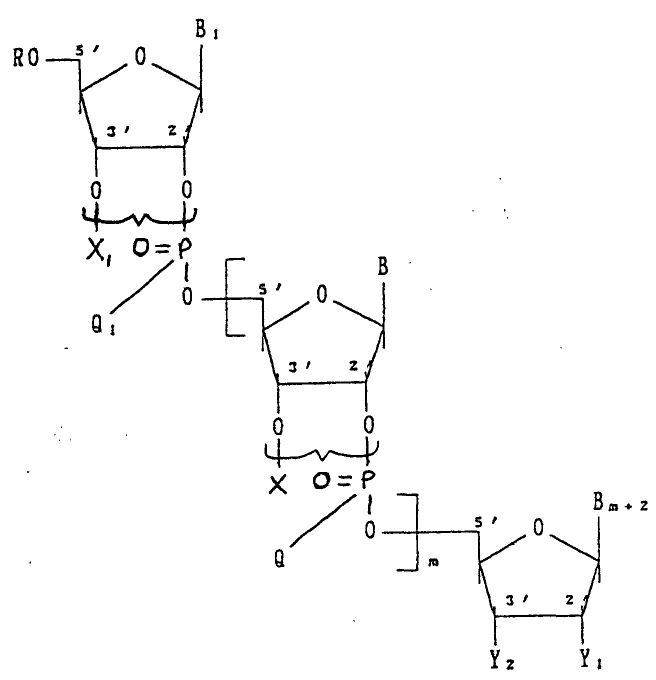 wherein:
m is an integer of 1 to 100;
B represents B₂, B₃, ..... B₍ₘ₊₁₎, and B₁ to B₍ₘ₊₂₎, which may be the same or different, each represents any one of hypoxanthine-9-yl, guanine-9-yl, adenine-9-yl, cytosine-1-yl, uracil-1-yl and thymine-1-yl;
Q represents Q₂, Q₃, ..... Q₍ₘ₊₁₎, and Q₁ to Q₍ₘ₊₁₎, independently represents any one of a thioanion, an oxoanion, an alkyl group, an aryloxy group, an alkylamino group, an arylamino group, an alkylthio group and an arylthio group (provided that at least one of Q₁ to Q₍ₘ₊₁₎ represents a thioanion) ;
X represents X₂, X₃, ..... X₍ₘ₊₁₎, and X₁ to X₍ₘ₊₁₎, which may be the same or different, each represents any one of a methyl group or an acyl group having 1 to 20 carbon atoms;
R represents a hydrogen atom, an acyl group having 1 to 20 carbon atoms, a phosphoryl group which may optionally have a substituent, a thiophosphoryl group which may optionally have a substituent or an alkylaminophosphoryl group which may optionally have a substituent; and
Y₁ and Y₂ independently represent a methoxy group, an acyloxy group having 1 to 20 carbon atoms, a hydrogen atom, a hydroxyl group, an alkyloxy group which may optionally have a substituent, a phosphoryloxy group which may optionally have a substituent, a thiophosphoryloxy group which may optionally have a substituent and an alkylaminophosphoryloxy group which may optionally have a substituent;
and wherein 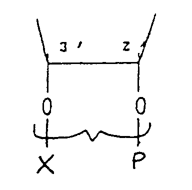 indicates that a group X is bound to an oxygen atom either at the 2' position or at the 3' position in the sugar moiety of the nucleoside monomer and that the phosphorus atom (P) is bound to an oxygen atom at either the 3' position or at the 2' position; respectively, in the sugar moiety of the nucleoside monomer, with the proviso that at least one of the groups X is bound to an oxygen atom at the 3' position.

2. A derivative as claimed in claim 1, wherein said substituent on R, Y₁ and Y₂, which may be the same or different, each represents any one of a cyanoethyl group, a chlorophenyl group, a monophosphoryl group, a pyrophosphoryl group, a hydrocarbon residue having 1 to 20 carbon atoms, a cholesteryl group and a group having the formula J-(K-O-L)_{E} wherein J represents: 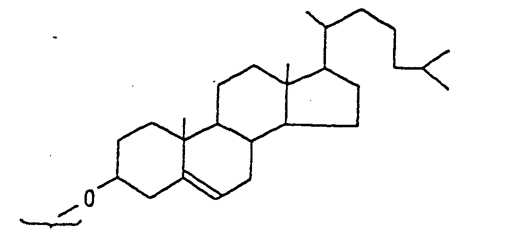 a hydroxyl group or any one of the oligoribonucleotidyl groups defined for the structural formula of said derivative according to claim 1, from which any one of RO, Y₁ and Y₂ is removed;
K represents any one of a phosphoryl group, a thiophosphoryl group and an alkylaminophosphoryl group having 1 to 10 carbon atoms;
L represents a hydrocarbon residue having 1 to 20 carbon atoms; and
E represents an integer of 1 to 10.

3. A derivative as claimed in claim 1, wherein m represents 25, B₁ to B₂₇ represents, in sequence, U, U, C, C, C, U, U, U, C, G, C, U, U, U, C, A, A, G, U, C, C, C, U, G, U, U, C and G; Q₁ to Q₂₆ each represent a thioanion; Y₁ and Y₂ represent OCH₃; and R represents H.

4. A derivative as claimed in claim 1, wherein m represents 28, B₁ to B₃₀ represent, in sequence U, C, C, U, U, C, U, A, G, C, U, C, C, G, C, U, A, G, U, C, A, A, A, A, U, U, U and U; Q₁ to Q₂₉ each represent a thioanion; Y₁ and Y₂ represent OCH₃; and R represents H.

5. A derivative as claimed in claim 1, wherein m represents 26, B₁ to B₂₈ represent, in sequence, U, U, U, U, A, A, U, U, U, A, U, A, U, U, U, U, U, U, C, U, U, U, C, C, C, C, C and U; Q₁ to Q₂₇ each represent a thioanion; Y₁ and Y₂ represent OCH₃; and R represents H.

6. A derivative as claimed in claim 1, having the structural formula: 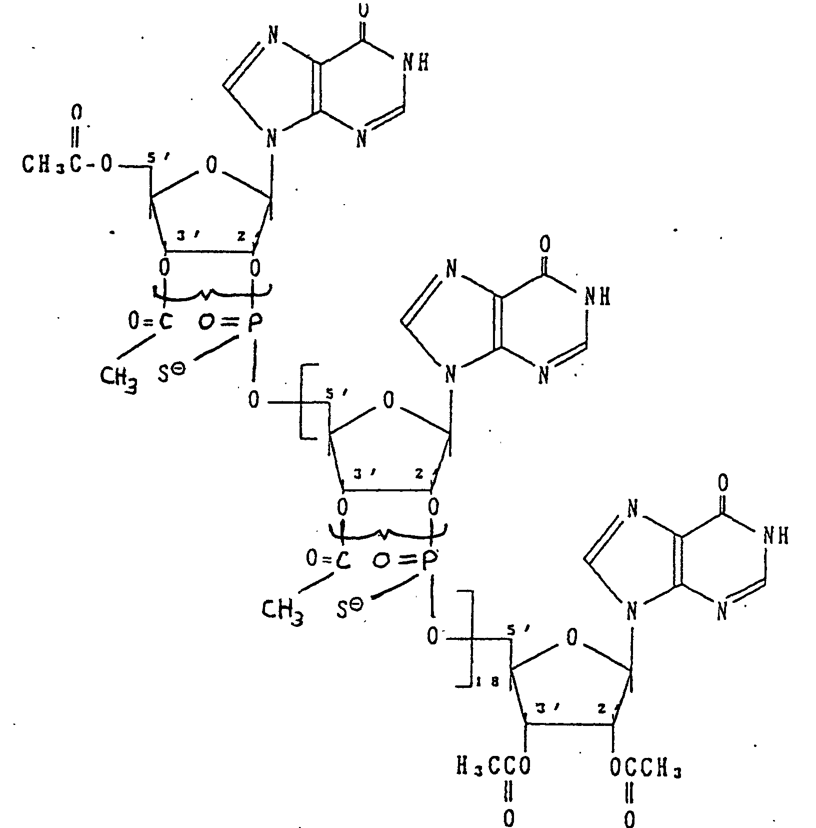 wherein 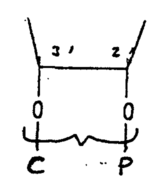 indicates that the acetyl group is bound to an oxygen atom either at the 2' position or at the 3' position in the sugar moiety of the nucleoside monomer and that the phosphorus atom (P) is bound to an oxygen atom at either the 3' position or at the 2' position, respectively, in the sugar moiety of the nucleoside monomer, with the proviso that at least one of the acetyl groups is bound to an oxygen atom at the 3' position.

7. An anti-AIDS viral agent comprising as an effective ingredient at least oligoribonucleolide derivative as claimed in any of claims 1 to 6.

8. An oligoribonucleotide derivative represented by the general formula: 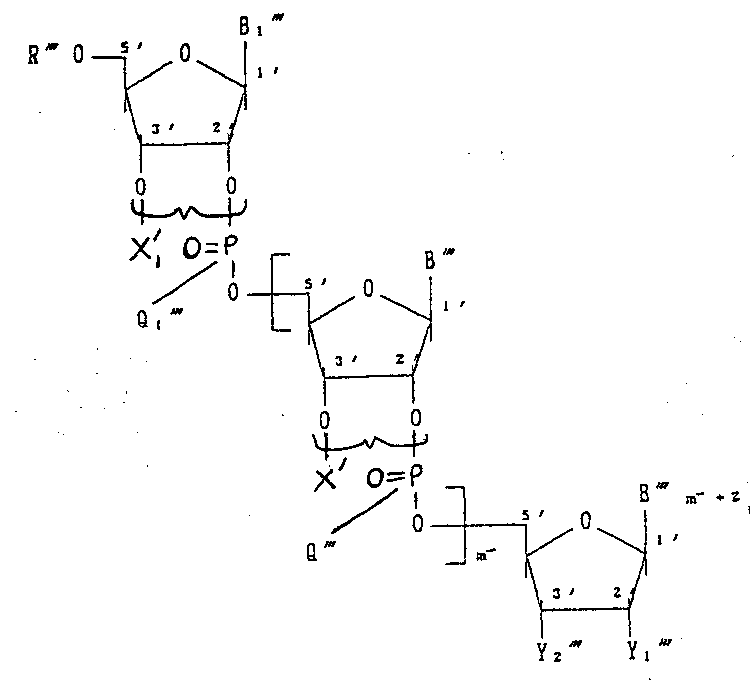 wherein:
m"' is an integer of 1 to 100;
B"' represents B"'₂, B"'₃, ..... B"'_{(m"'+1)}, and B"'₁ to B"'_{(m"'+2)} which may be the same or different, each represents any one of hypoxanthine-9-yl, guanine-9-yl, adenine-9-yl, cytosine-1-yl, uracil-1-yl and thymine-1-yl (provided that all of them do not represent adenine-9-yl);
Q" represents Q"'₂, Q"'₃, ..... Q"'_{(m"'+1)}, and Q"'₁ to Q"'_{(m"'+1)}, independently represents any one of a thioanion, an oxoanion, an aryloxy group, an alkylamino group, an arylamino group, an alkylthio group and an arylthio group (provided that at least one of Q"'₁ to Q"'_{(m"'+1)}, represents a thioanion) ;
X' represents X'₂, X'₃, ..... X'_{(m"'+1)}, and X'₁ to X'_{(m"'+1)} each represents a methyl group;
R"' represents any one of a hydrogen atom, an acyl group having 1 to 20 carbon atoms and a phosphoryl group which may optionally have a substituent; and
Y"'₁ and Y"'₂ independently represents any one of a methoxy group, an acyloxy group having 1 to 20 carbon atoms, an alkyloxy group which may optionally have a substituent, a hydroxyl group, an alkylsilyl group, a hydrogen atom and a phosphoryl group which may optionally have a substituent; and wherein 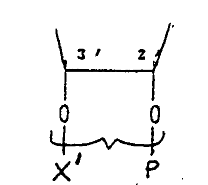 indicates that a group X' is bound to an oxygen atom either at the 2' position or at the 3' position in the sugar moiety of the nucleoside monomer and that the phosphorus atom (P) is bound to an oxygen atom at either the 3' position or at the 2' position, respectively, in the sugar moiety of the nucleoside monomer, with the proviso that at least one of the groups X' is bound to an oxygen atom at the 3' position.

9. A derivative as claimed in claim 8, wherein said substituent is any one of a cyanoethyl group, a chlorophenyl group and a hydrocarbon residue having 1 to 20 carbon atoms.

10. A derivative as claimed in claim 8, having the structural formula: 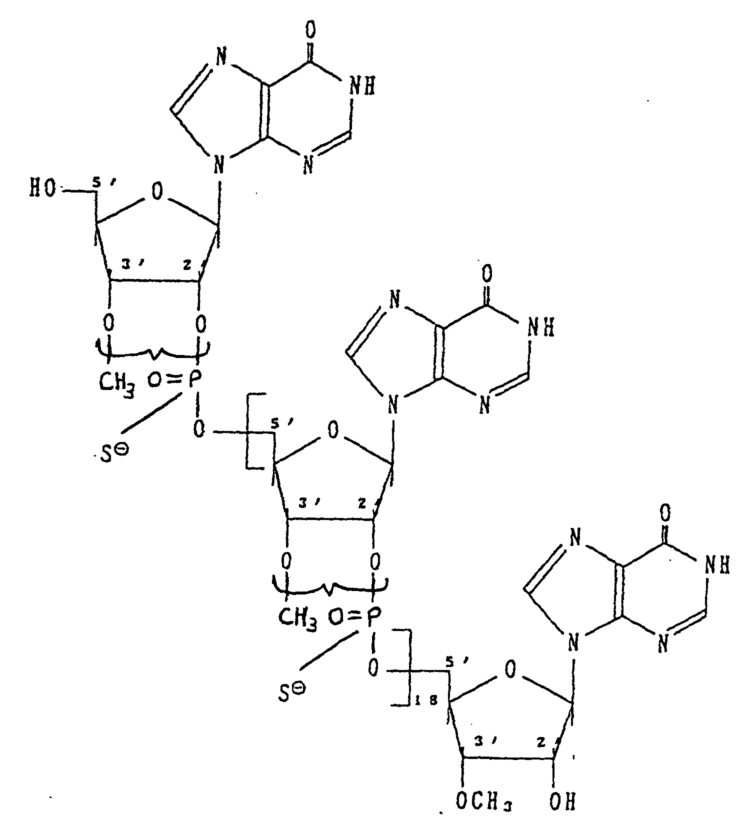 and wherein 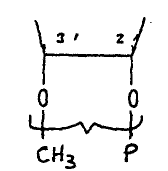 indicates that a group CH₃ is bound to an oxygen atom either at the 2' position or at the 3' position in the sugar moiety of the nucleoside monomer and that the phosphorus atom (P) is bound to an oxygen atom at either the 3' position or at the 2' position, respectively, in the sugar moiety of the nucleoside monomer, with the proviso that at least one of the groups CH₃ is bound to an oxygen atom at the 3' position.

## Revendications

1. Dérivé d'oligoribonucléotide représenté par la formule générale : 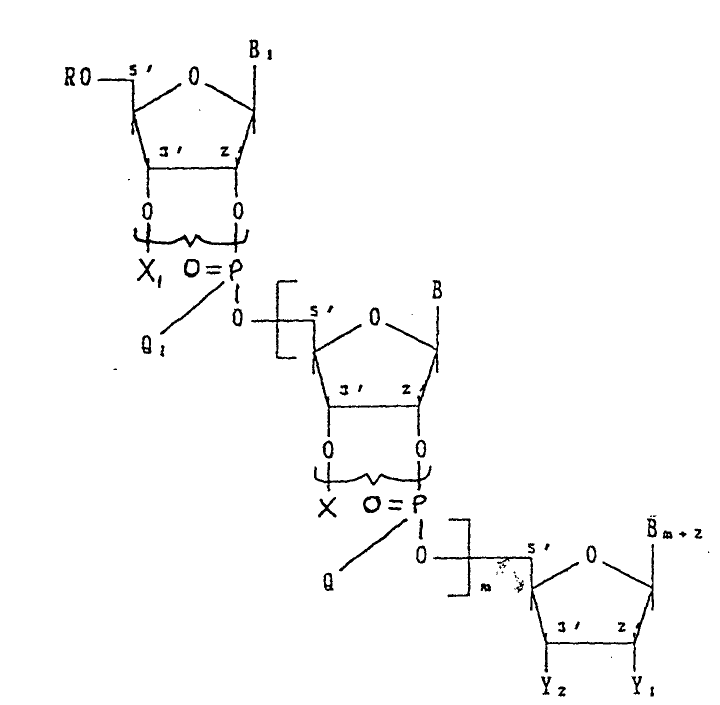 dans laquelle :
m représente un nombre entier de 1 à 100 ;
B représente B₂, B₃, ... B₍ₘ₊₁₎, et B₁ à B₍ₘ₊₂₎, qui peuvent être identiques ou différents, représentent chacun l'un quelconque des groupes hypoxanthine-9-yle, guanine-9-yle, adénine-9-yle, cytosine-1-yle, uracile-1-yle et thymine-1-yle ;
Q représente Q₂, Q₃, ... Q₍ₘ₊₁₎, et Q₁ à Q₍ₘ₊₁₎ représentent, indépendamment, l'une quelconque des entités consistant en un thioanion, un oxanion, un groupe alkyle, un groupe aryloxy, un groupe alkylamino, un groupe arylamino, un groupe alkylthio et un groupe arylthio (sous réserve qu'au moins un des groupes Q₁ à Q₍ₘ₊₁₎ représente un thioanion) ;
X représente X₂, X₃, ... X₍ₘ₊₁₎, et X₁ à X₍ₘ₊₁₎, qui peuvent être identiques ou différents, représentent chacun l'un quelconque des groupes consistant en un groupe méthyle et un groupe acyle ayant 1 à 20 atomes de carbone ;
R représente un atome d'hydrogène, un groupe acyle ayant 1 à 20 atomes de carbone, un groupe phosphoryle qui peut porter facultativement un substituant, un groupe thiophosphoryle qui peut porter facultativement un substituant ou un groupe alkylaminophosphoryle qui peut porter facultativement un substituant ; et
Y₁ et Y₂ représentent, indépendamment, un groupe méthoxy, un groupe acyloxy ayant 1 à 20 atomes de carbone, un atome d'hydrogène, un groupe hydroxyle, un groupe alkyloxy qui peut porter facultativement un substituant, un groupe phosphoryloxy qui peut porter facultativement un substituant, un groupe thiophosphoryloxy qui peut porter facultativement un substituant et un groupe alkylaminophosphoryloxy qui peut porter facultativement un substituant ;
et dans laquelle 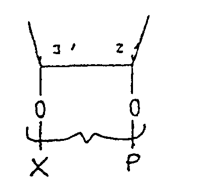 indique qu'un groupe X est lié à un atome d'oxygène en position 2' ou en position 3' dans le groupement glucidique du monomère nucléoside et que l'atome de phosphore (P) est lié à un atome d'oxygène en position 3' ou en position 2', respectivement, dans le groupement glucidique du monomère nucléoside, sous réserve qu'au moins un des groupes X soit lié à un atome d'oxygène en position 3'.

2. Dérivé suivant la revendication 1, dans lequel les substituants sur R, Y₁ et Y₂, qui peuvent être identiques ou différents, représentent chacun l'un quelconque des groupes consistant en un groupe cyano-éthyle, un groupe chlorophényle, un groupe monophosphoryle, un groupe pyrophosphoryle, un résidu hydrocarboné ayant 1 à 20 atomes de carbone, un groupe cholestéryle et un groupe répondant à la formule J-(K-O-L)_{E} dans laquelle J représente un groupe : 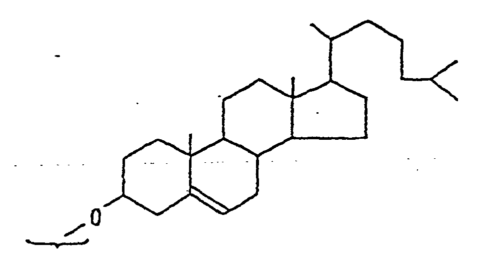 un groupe hydroxyle ou l'un quelconque des groupes oligoribonucléotidyle définis pour la formule structurale dudit dérivé suivant la revendication 1, duquel l'un quelconque des groupes RO, Y₁ et Y₂ est éliminé ;
K représente l'un quelconque des groupes consistant en un groupe phosphoryle, un groupe thiophosphoryle et un groupe alkylaminophosphoryle ayant 1 à 10 atomes de carbone ;
L représente un résidu hydrocarboné ayant 1 à 20 atomes de carbone ; et
E représente un nombre entier de 1 à 10.

3. Dérivé suivant la revendication 1, dans lequel m est égal à 25, B₁ à B₂₇ représentent, en séquence, U, U, C, C, C, U, U, U, C, G, C, U, U, U, C, A, A, G, U, C, C, C, U, G, U, U, C et G ; Q₁ à Q₂₆ représentent chacun un thioanion ; Y₁ et Y₂ représentent un groupe OCH₃ ; et R représente H.

4. Dérivé suivant la revendication 1, dans lequel m est égal à 28, B₁ à B₃₀ représentent, en séquence, U, C, C, U, U, C, U, A, G, C, U, C, C, G, C, U, A, G, U, C, A, A, A, A, U, U, U et U ; Q₁ à Q₂₉ représentent chacun un thioanion ; Y₁ et Y₂ représentent un groupe OCH₃ ; et R représente H.

5. Dérivé suivant la revendication 1, dans lequel m est égal à 26, B₁ à B₂₈ représentent, en séquence, U, U, U, U, A, A, U, U, U, A, U, A, U, U, U, U, U, U, C, U, U, U, C, C, C, C, C et U ; Q₁ à Q₂₇ représentent chacun un thioanion ; Y₁ et Y₂ représentent un groupe OCH₃ ; et R représente H.

6. Dérivé suivant la revendication 1, répondant à la formule structurale : 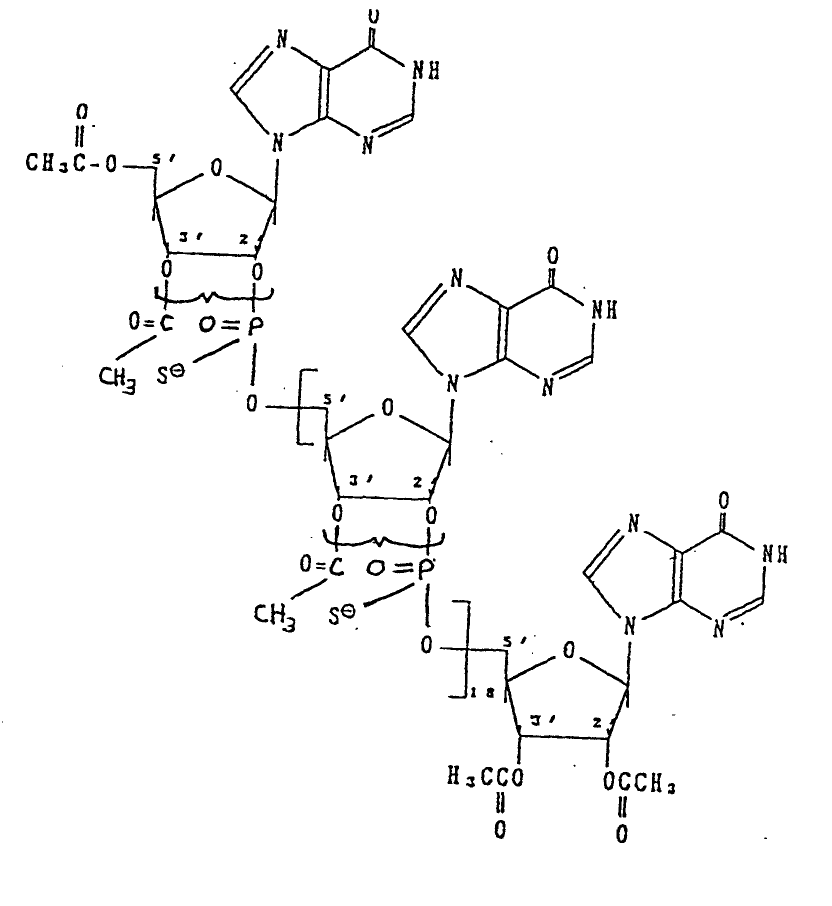 dans laquelle 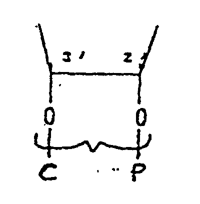 indique que le groupe acétyle est lié à un atome d'oxygène en position 2' ou en position 3' dans le groupement glucidique du monomère nucléoside et que l'atome de phosphore (P) est lié à un atome d'oxygène en position 3' ou en position 2', respectivement, dans le groupement glucidique du monomère nucléoside, sous réserve qu'au moins un des groupes acétyle soit lié à un atome d'oxygène en position 3'.

7. Agent anti-virus du SIDA, comprenant, comme ingrédient efficace, au moins un dérivé d'oligoribonucléotide suivant l'une quelconque des revendications 1 à 6.

8. Dérivé d'oligoribonucléotide représenté par la formule générale : 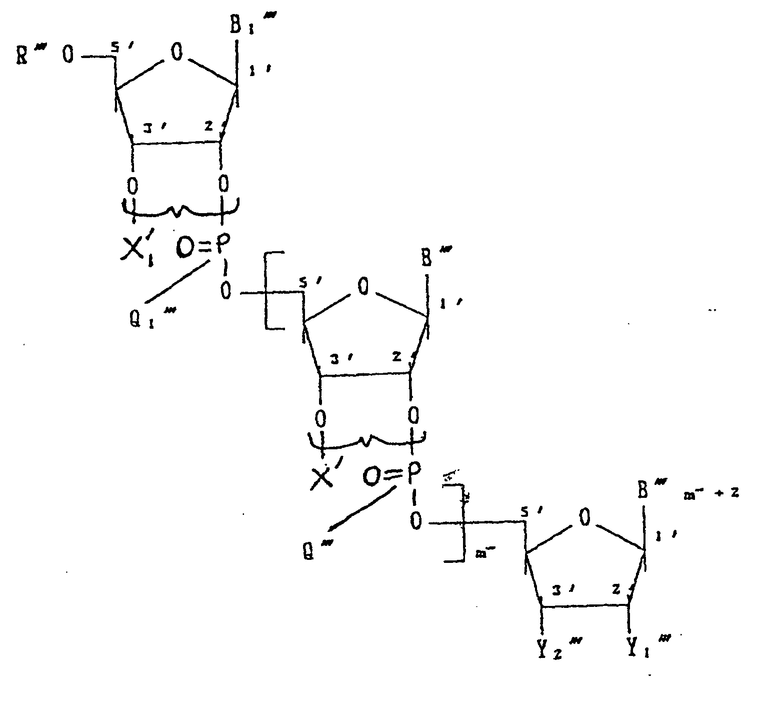 dans laquelle :
m''' représente un nombre entier de 1 à 100 ;
B''' représente B'''₂, B'''₃, ... B'''₍ₘ₊₁₎, et B'''₁ à B'''_{(m'''+2)}, qui peuvent être identiques ou différents, représentent chacun l'un quelconque des groupes hypoxanthine-9-yle, guanine-9-yle, adénine-9-yle, cytosine-1-yle, uracile-1-yle et thymine-1-yle (sous réserve que la totalité de ces groupes ne représente pas des groupes adénine-9-yle) ;
Q" représente Q'''₂, Q'''₃, ... Q'''_{(m'''+1)}, et Q'''₁ à Q'''_{(m'''+1)} représentent, indépendamment, l'une quelconque des entités consistant en un thioanion, un oxanion, un groupe aryloxy, un groupe alkylamino, un groupe arylamino, un groupe alkylthio et un groupe arylthio (sous réserve qu'au moins un des groupes Q'''₁ à Q'''_{(m'''+1)} représente un thioanion) ;
X' représente X'₂, X'₃, ... X'_{(m'''+1)}, et X'₁ à X'_{(m'''+1)} représentent chacun un groupe méthyle ;
R''' représente l'une quelconque des entités consistant en un atome d'hydrogène, un groupe acyle ayant 1 à 20 atomes de carbone et un groupe phosphoryle qui peut porter facultativement un substituant ; et
Y'''₁ et Y'''₂ représentent, indépendamment, l'un quelconque des groupes consistant en un groupe méthoxy, un groupe acyloxy ayant 1 à 20 atomes de carbone, un groupe alkyloxy qui peut porter facultativement un substituant, un groupe hydroxyle, un groupe alkylsilyle, un atome d'hydrogène et un groupe phosphoryle qui peut porter facultativement un substituant ;
et dans laquelle 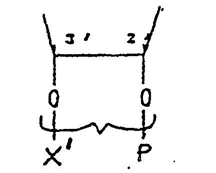 indique qu'un groupe X' est lié à un atome d'oxygène en position 2' ou en position 3' dans le groupement glucidique du monomère nucléoside et que l'atome de phosphore (P) est lié à un atome d'oxygène en position 3' ou en position 2', respectivement, dans le groupement glucidique du monomère nucléoside, sous réserve qu'au moins un des groupes X' soit lié à un atome d'oxygène en position 3'.

9. Dérivé suivant la revendication 8, dans lequel le substituant est l'un quelconque des groupes consistant en un groupe cyano-éthyle, un groupe chlorophényle et un résidu hydrocarboné ayant 1 à 20 atomes de carbone.

10. Dérivé suivant la revendication 8, répondant à la formule structurale : 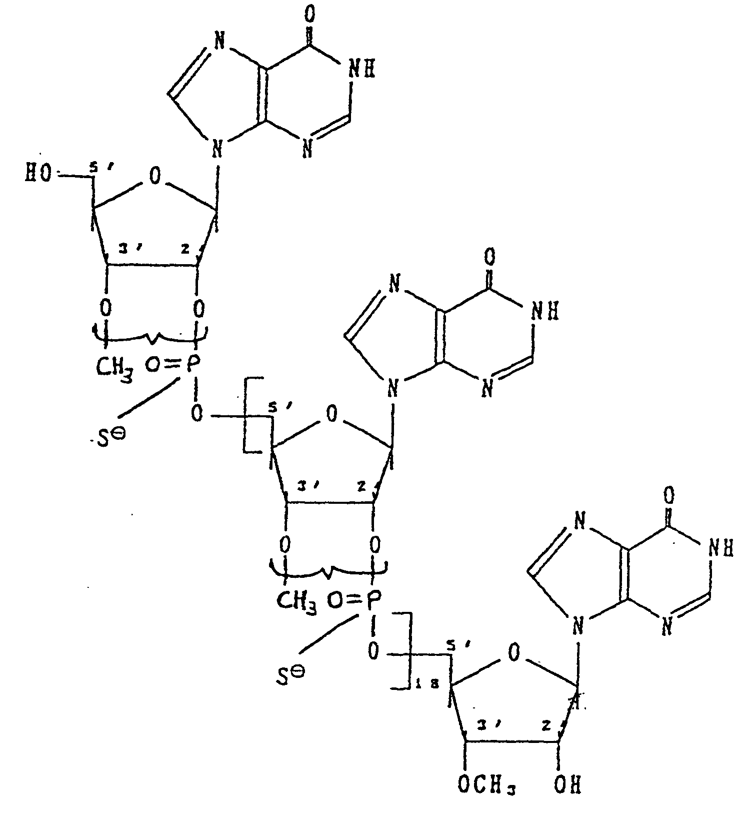 dans laquelle 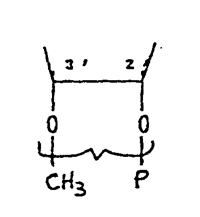 indique qu'un groupe CH₃ est lié à un atome d'oxygène en position 2' ou en position 3' dans le groupement glucidique du monomère nucléoside et que l'atome de phosphore (P) est lié à un atome d'oxygène en position 3' ou en position 2', respectivement, dans le groupement glucidique du monomère nucléoside, sous réserve qu'au moins un des groupes CH₃ soit lié à un atome d'oxygène en position 3'.

## Patentansprüche

1. Oligoribonucleotid-Derivat der allgemeinen Formel: 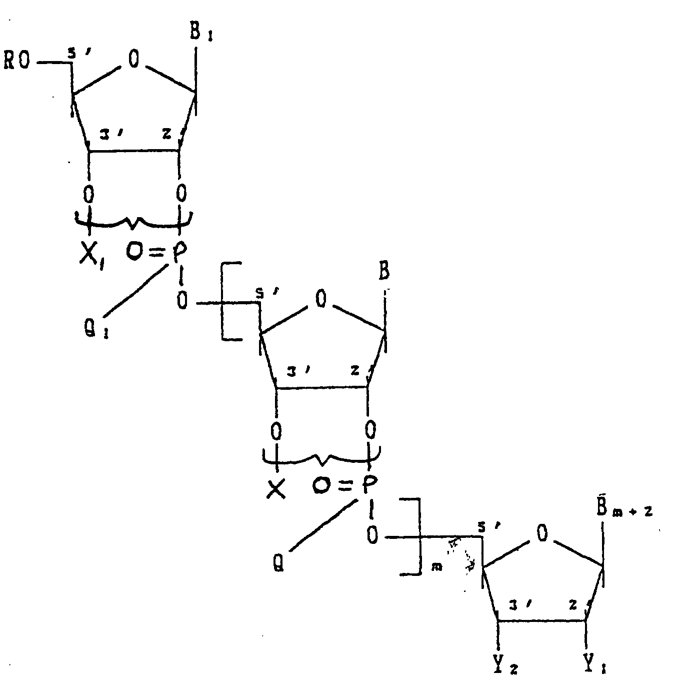 worin
m eine ganze Zahl von 1 bis 100 ist;
B gleich B₂, B₃, ... B₍ₘ₊₁₎ und B₁ bis B₍ₘ₊₂₎, die gleich oder verschieden sein können, jeweils Hypoxanthin-9-yl, Guanin-9-yl, Adenin-9-yl, Cytosin-1-yl, Uracil-1-yl and Thymin-1-yl;
Q gleich Q₂, Q₃, ... Q₍ₘ₊₁₎ und Q₁ bis Q₍ₘ₊₁₎ unabhängig voneinander Thioanion, Oxoanion, Alkylgruppe, Aryloxygruppe, Alkylaminogruppe, Arylaminogruppe, Alkylthiogruppe oder Arylthiogruppe, vorausgesetzt, mindestens ein Q₁ bis Q₍ₘ₊₁₎ ist ein Thioanion;
X gleich X₂, X₃, ... X₍ₘ₊₁₎ und X₁ bis X₍ₘ₊₁₎, die gleich oder verschieden sein können, jeweils Methylgruppe oder Acylgruppe mit 1 bis 20 Kohlenstoffatomen;
R gleich Wasserstoffatom, Acylgruppe mit 1 bis 20 Kohlenstoffatomen, Phosphorylgruppe, die wahlfrei einen Substituenten tragen kann, Thiophosphorylgruppe, die wahlfrei einen Substituenten tragen kann, oder Alkylaminophosphorylgruppe, die wahlfrei einen Substituenten tragen kann; und
Y₁ und Y₂ voneinander unabhängig gleich Methoxygruppe, Acyloxygruppe mit 1 bis 20 Kohlenstoffatomen, Wasserstoffatom, Hydroxylgruppe, Alkyloxygruppe, die wahlfrei einen Substituenten tragen kann, Phosphoryloxygruppe, die wahlfrei einen Substituenten tragen kann, Thiophosphoryloxygruppe, die wahlfrei einen Substituenten tragen kann, oder Alkylaminophosphoryloxygruppe, die wahlfrei einen Substituenten tragen kann,
worin 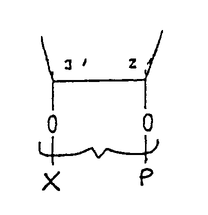 bedeutet, dass die X-Gruppe am Sauerstoffatom entweder in 2' - oder 3'-Stellung am Zuckerrest des Nucleosidmonomers gebunden ist und dass das Phosphoratom (P) am anderen Sauerstoffatom in 3'- bzw. in 2'-Stellung am Zuckerrest des Nucleosidmonomers gebunden ist, vorausgesetzt, mindestens eine der X-Gruppen ist am Sauerstoffatom in der 3'-Stellung gebunden.

2. Derivat nach Anspruch 1, wobei der Substituent an R, Y₁ und Y₂, welche gleich oder ungleich sein können, jeweils ist aus Cyanoethylgruppe, Chlorphenylgruppe, Monophosphorylgruppe, Pyrophosphorylgruppe, Kohlenwasserstoffrest mit 1 bis 20 Kohlenstoffatomen, Cholesterylgruppe und Gruppe der Formel J-(K-O-L)_{E}, worin J steht für 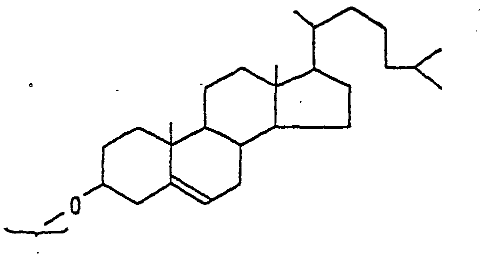 eine Hydroxylgruppe oder eine der Oligoribonucleotidgruppen mit der Derivatstrukturformel von Anspruch 1, in der eines der RO, Y₁ und Y₂ entfernt ist;
K für Phosphorylgruppe, Thiophosphorylgruppe oder Alkylaminophosphorylgruppe mit 1 bis 10 Kohlenstoffatomen;
L für Kohlenwasserstoffrest mit 1 bis 20 Kohlenstoffatomen; und
E für eine ganze Zahl von 1 bis 10.

3. Derivat nach Anspruch 1, wobei m für 25 steht, B₁ bis B₂₇ für nacheinander U, U, C, C, C, U, U, U, C, G, C, U, U, U, C, A, A, G, U, C, C, C, U, G, U, U, C und G; Q₁ bis Q₂₆ jeweils für Thioanion, Y₁ und Y₂ für OCH₃, und R für H.

4. Derivat nach Anspruch 1, wobei m für 28 steht, B₁ bis B₃₀ für nacheinander U, C, C, U, U, C, U, A, G, C, U, C, C, G, C, U, A, G, U, C, A, A, A, A, U, U, U und U; Q₁ bis Q₂₉ jeweils für Thioanion, Y₁ und Y₂ für OCH₃ und R für H.

5. Derivat nach Anspruch 1, wobei m für 26 steht, B₁ bis B₂₈ für nacheinander U, U, U, U, A, A, U, U, U, A, U, A, U, U, U, U, U, U, C, U, U, U, C, C, C, C, C und U; Q₁ bis Q₂₇ jeweils für Thioanion, Y₁ und Y₂ für OCH₃ und R für H.

6. Derivat nach Anspruch 1 mit der Strukturformel: 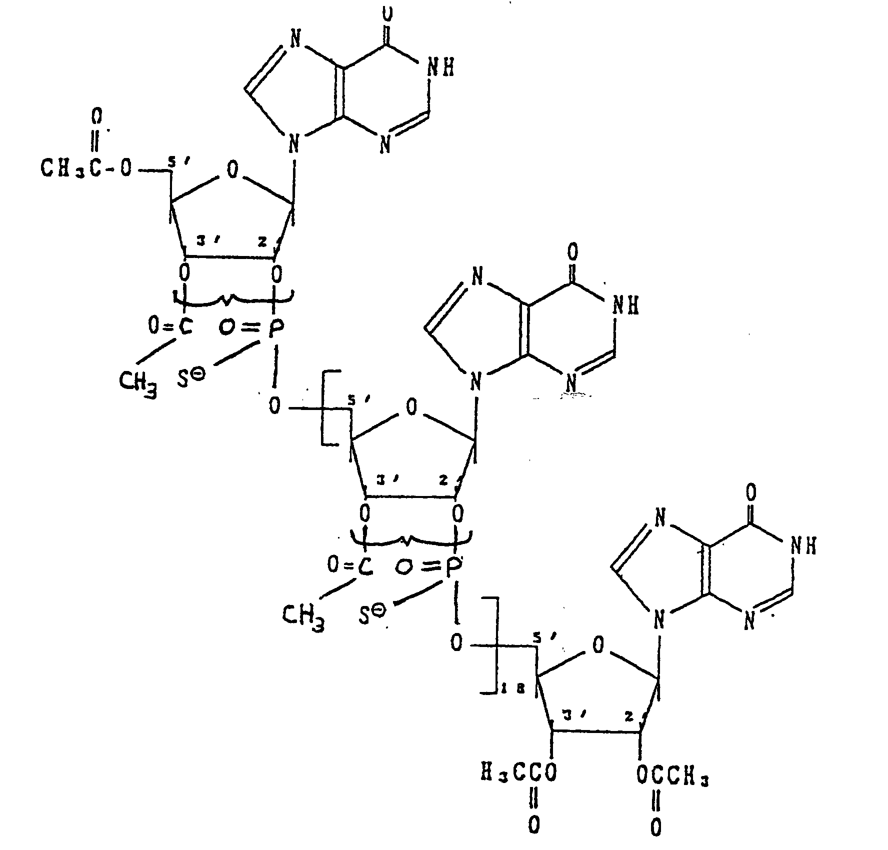 worin 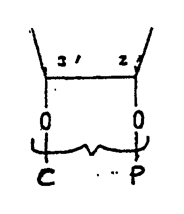 bedeutet, dass die Acetylgruppe am Sauerstoffatom entweder in 2' - oder 3'-Stellung am Zuckerrest des Nucleosidmonomers gebunden ist, und dass das Phosphoratom (P) am Sauerstoffatom in 3'- bzw. 2'-Stellung am Zuckerrest des Nucleosidmonomers gebunden ist, vorausgesetzt, mindestens eine der Acetylgruppen ist am Sauerstoffatom in der 3'-Stellung gebunden.

7. Anti-AIDS-Virusmittel, das als Wirkbestandteil mindestens ein Oligoribonucleotid-Derivat nach einem der Ansprüche 1 bis 6 enthält.

8. Oligoribonucleotid-Derivat nach der allgemeinen Formel: 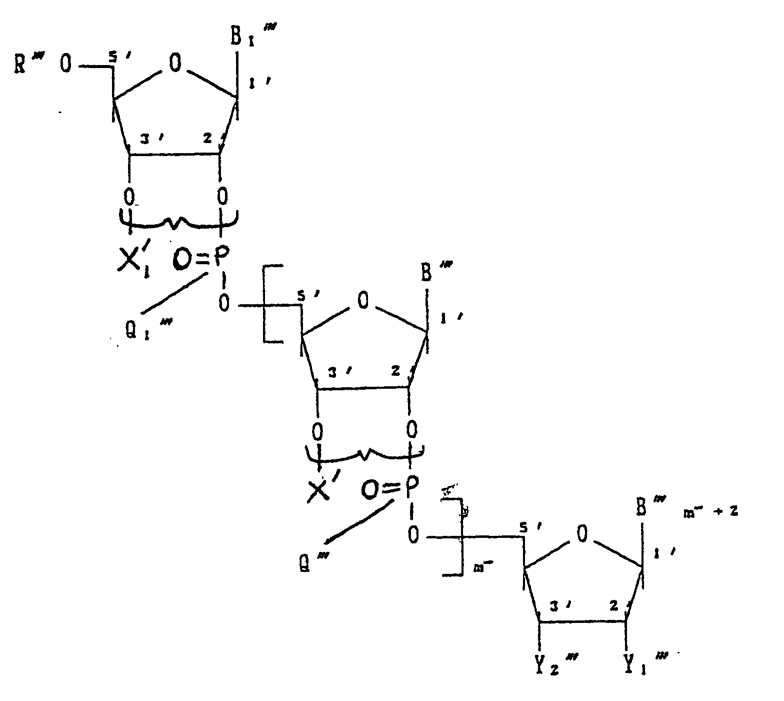 worin ist:
m''' eine ganze Zahl von 1 bis 100;
B''' gleich B'''₂, B'''₃, ... B'''_{(m'''+1)} und B'''₁ bis B'''_{(m'''+2)}, welche gleich oder verschieden sein können, jeweils Hypoxanthin-9-yl, Guanin-9-yl, Adenin-9-yl, Cytosin-1-yl, Uracil-1-yl und Thymin-1-yl, vorausgesetzt, es sind nicht alle Adenin-9-yl;
Q'' gleich Q'''₂, Q'''₃, ... Q'' '_{(m'''+1)}, und Q'''₁ bis Q'''_{(m'''+1)} jeweils unabhängig voneinander Thioanion, Oxoanion, Aryloxygruppe, Alkylaminogruppe, Arylaminogruppe, Alkylthiogruppe und Arylthiogruppe, vorausgesetzt, mindestens ein Q'''₁ bis Q'''_{(m'''+1)} ist ein Thioanion;
X' gleich X'₂, X'₃, ... X'_{(m'''-1)} und X'₁ bis X'_{(m'''+1)} jeweils eine Methylgruppe;
R''' gleich Wasserstoffatom, Acylgruppe mit 1 bis 20 Kohlenstoffatomen oder Phosphorylgruppe, die wahlfrei einen Substituenten tragen kann; und
Y'''₁ und Y'''₂ unabhängig voneinander gleich Methoxygruppe, Acyloxygruppe mit 1 bis 20 Kohlenstoffatomen, Alkyloxygruppe, die wahlfrei einen Substituenten tragen kann, Hydroxylgruppe, Alkylsilylgruppe, Wasserstoffatom oder Phosphorylgruppe, die wahlfrei einen Substituenten tragen kann;
und worin 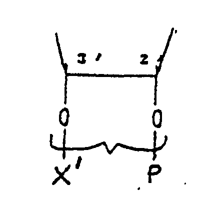 bedeutet, dass eine Gruppe X' am Sauerstoffatom entweder in der 2'- oder 3'-Stellung am Zuckerrest des Nucleosidmonomers gebunden ist, und dass das Phosphoratom (P) am jeweils anderen Sauerstoffatom in 3'- bzw. 2'-Stellung am Zuckerrest des Nucleosidmonomers gebunden ist, vorausgesetzt, mindestens eine der X'-Gruppen ist am Sauerstoffatom in der 3'-Stellung gebunden.

9. Derivat nach Anspruch 8, wobei der Substituent eine Cyanoethylgruppe, eine Chlorophenylgruppe oder ein Kohlenwasserstoffrest mit 1 bis 20 Kohlenstoffatomen ist.

10. Derivat nach Anspruch 8 mit der Strukturformel: 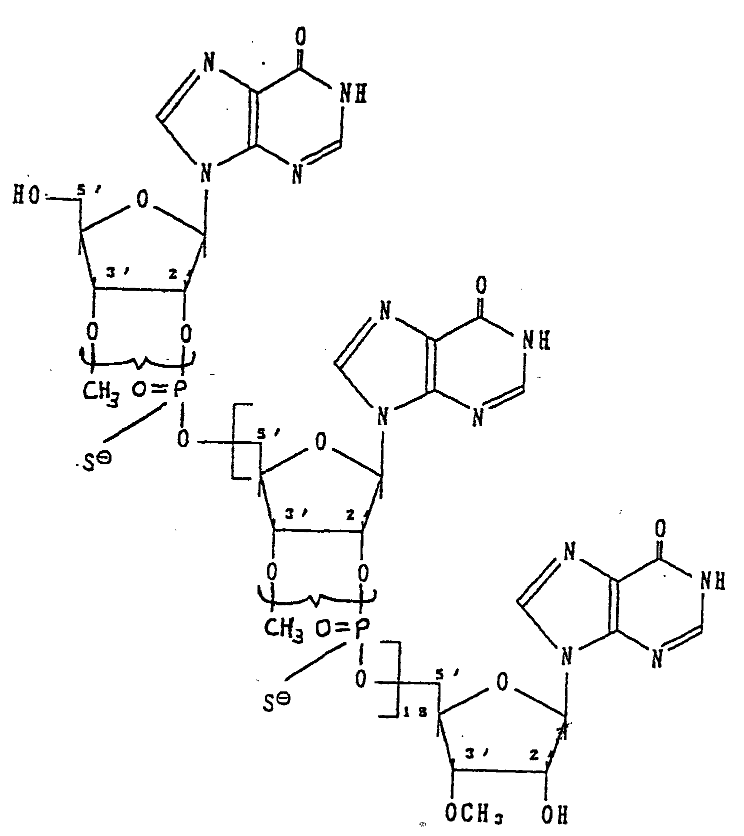 worin 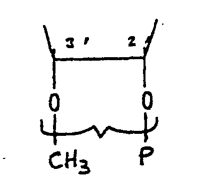 bedeutet, dass die Gruppe CH₃ am Sauerstoffatom entweder in der 2'- oder in der 3'-Stellung am Zuckerrest des Nucleosidmonomers gebunden ist, und dass das Phosphoratom (P) am jeweils anderen Sauerstoffatom in 3'- bzw. 2'-Stellung am Zuckerrest des Nucleosidmonomers gebunden ist, vorausgesetzt, mindestens eine der CH₃-Gruppen ist am Sauerstoffatom in der 3'-Stellung gebunden.
